# FASCICULE DE BREVET EUROPEEN

(11) **EP 2 367 926 B1**
(45) Date de publication et mention de la délivrance du brevet: **06.03.2013**
(21) Numéro de dépôt: 09804286.4
(22) Date de dépôt: 22.12.2009
(51) Int. Cl.: C12M 1/00, E04B 1/92

(54) **DISPOSITIF DE CULTURE D'ALGUES ET/OU DE MICROORGANISMES POUR LE TRAITEMENT D'UN EFFLUENT ET BIOFAÇADE**
VORRICHTUNG ZUR KULTIVIERUNG VON ALGEN UND/ODER MIKROORGANISMEN ZUR ABWASSERBEHANDLUNG UND BIOLOGISCHE FASSADE
Device for cultivating algae and/or microorganisms for treating an effluent and biological façade

(30) Priorité: 23.12.2008 EP 08291237
(43) Date de publication de la demande: 28.09.2011
(73) Titulaire: X'TU, 75010 Paris (FR)
(72) Inventeur: LEGENDRE, Anouk, F-75010 Paris (FR); DESMAZIERES, Nicolas, F-75010 Paris (FR)
(74) Mandataire: Novagraaf Technologies
(86) Numéro de dépôt international: PCT/FR2009/001474
(87) Numéro de publication internationale: WO 2010/072925

(56) Documents cités:
- WO-A1-2005/001104
- DE-A1- 3 607 864
- GB-A- 2 320 031
- US-A- 5 741 702
- US-A1- 2005 260 553
- US-A1- 2007 264 708
- US-B1- 6 399 367
- DATABASE WPI Week 200122 Thomson Scientific, London, GB; AN 2001-213304 XP002585042 & JP 2000 320041 A (MAEDA KENSETSU KOGYO KK) 21 novembre 2000 (2000-11-21)

## Description

### Domaine technique

La présente invention se rapporte à l'utilisation d'un dispositif permettant de traiter des effluents et/ou par exemple de faire produire aux façades de bâtiments de l'énergie primaire biochimique obtenue notamment par photosynthèse. L'utilisation selon la présente invention permet notamment de fabriquer du biocarburant, des molécules organiques, des composés chimiques, des protéines.

Le biocarburant ainsi obtenu, peut être par exemple de la biomasse oléagineuse directement utilisable en centrale thermique ou transformable par pyrolyse en charbon ou en biopétrole.

L'invention peut être utilisée directement pour les besoins énergétiques du bâtiment et alimenter sa propre centrale énergétique et/ou être exporter et /ou vendu.

La présente invention permet en outre de mettre en oeuvre de nouvelles biofaçades et bio réacteurs Intégrés en façades de bâtiments modernes ou anciens, en construction ou existant déjà.

### Etat de la technique

Les villes polluent et produisent beaucoup de gaz carbonique par leurs centrales thermiques, par les chaufferies des bâtiments, dans les centrales de production de froid. Par ailleurs, le traitement de l'air vicié des bâtiments n'est pas toujours évident, notamment lorsqu'il est issu des humains, mais aussi des parkings souterrains, notamment des automobiles.

En particulier, du gaz carbonique (CO₂) et de l'oxyde d'azote (NO₂) s'échappent dans l'atmosphère et contribue à l'effet de serre et à la dégradation du climat.

Les villes consomment aussi beaucoup d'énergie, qui est produite à distance, et doit être transportée à grands frais avec une importante perte de charge même pour l'énergie électrique. Les quartiers d'affaires intégralement climatisés et fonctionnant à l'énergie électrique via des centrales thermiques sont un gouffre à énergie.

De nombreuses solutions ont été étudiées pour traiter ces gaz, appelés effluents dans la présente, il s'agit notamment de filtres, de système d'épuration de l'air par des plantes, etc..

Malheureusement, ces systèmes sont donc très coûteux, difficiles à mettre en oeuvre, notamment sur des bâtiments existants, nécessitent un entretien complexe, lui même coûteux, et génèrent d'autres polluants, par exemples avec les filtres. Pour les systèmes avec des plantes, il y a aussi les problèmes du renouvellement et de l'entretien des plantes, qui sont compliqués, demandent beaucoup de mains d'oeuvre et sont difficilement automatisable.

Il existe donc un réel besoin d'un système palliant ces défauts, inconvénients et obstacles de l'art antérieur.

### Description de l'invention

La présente invention a précisément pour but de répondre aux besoins et inconvénients précités de l'art antérieur. La présente invention se rapporte notamment à l'utilisation d'un dispositif pour le traitement d'un effluent et/ou la fabrication d'une biomasse comprenant :
- un contenant de culture d'algues et/ou de microorganismes en milieu aqueux,
- un moyen d'alimentation de la culture d'algues et/ou de microorganismes
- un moyen d'injection dans la culture d'algues et/ou de microorganismes d'un effluent, ledit effluent provenant d'un bâtiment,
- un moyen de régulation de la température de la culture d'algues et/ou de microorganismes,
- éventuellement un éclairage favorable à la culture d'algues et/ou de microorganisme, et
- un moyen de récupération dudit effluent permettant de récupérer ledit effluent issu d'un bâtiment pour les injecter dans la culture d'algues et/ou de microorganismes,
dans lequel le contenant de culture d'algues et/ou de microorganismes est positionné entre deux surfaces : une surface de bâtiment et une surface extérieure formant une double peau, et ledit effluant provient du bâtiment possédant la double peau et/ou d'un autre bâtiment.

Le document WO 2005/001104 décrit une installation pour le traitement d'un effluant gazeux provenant d'un bâtiment.

Le document JP 2000320041 décrit des bâtiments avec des façades couvertes de plaques de bioréacteur.

Le document US 5 741 702 décrit un contenant de culture d'algues et/ou de microorganismes en milieu aqueux pouvant comprendre une face isolante.

L'objet de la présente invention est l'utilisation de ce dispositif pour le traitement d'un effluent et/ou la fabrication d'une biomasse.

Selon l'invention, par « bâtiment » on entend toute construction servant à loger des hommes, des animaux ou des choses. Il peut s'agir par exemple d'une construction, d'un bâtiment industriel et/ou de bureaux et/ou d'habitation et/ou agricole, par exemple une maison, un immeuble, une centrale thermique et/ou d'un ouvrage d'art souterrain, par exemple une infrastructure souterraine de circulation automobiles et/ou ferroviaire, par exemple un tunnel autoroutier, un tunnel de métro, un parking, un tunnel, une voierie souterraine, un espace sous dalles, une caverne ou grotte aménagée en habitat humain, animal, de culture ou pour utilisation industrielle ou de stockage.

Selon l'invention, l'effluent peut être, par exemple, un effluent gazeux ou un effluent liquide.

Selon l'invention, par « effluent liquide » on entend un effluent seul ou un mélange d'effluents liquides. Il peut s'agir, par exemple de tout liquide et/ou solution vicié issu d'un bâtiment. L'effluent liquide peut être vicié par l'occupation humaine. Il peut s'agir, par exemple, d'eaux usées issues des sanitaires, d'un liquide contenant un contaminant par exemple un métal, par exemple du plomb, du nickel, une substance polluante, par exemple des nitrates, des sels.

La présente invention permet notamment de retraiter les effluents liquides pollués, par exemple ceux précités, par exemple aussi des effluents avec des métaux, sels, composés chimiques, et autres polluants qui peuvent être, par exemple, évacués des bâtiments.

Selon l'invention, le traitement de l'effluent liquide un ou du mélange d'effluents liquides peut consister, par exemple, à éliminer, c'est-à-dire extraire, de l'effluent liquide au moins un polluant, un contaminant par substance polluante. Il peut par exemple consister à désaliniser un effluent liquide et/ou à renouveler le liquide traité. Le traitement peut être fonction de l'algue et/ou du microorganisme choisie. Selon l'invention, l'algue et / ou le microorganisme peut être choisi en fonction du traitement souhaité de l'effluent à traiter.

Dans la présente, par « effluent gazeux », on entend un seul effluent gazeux ou un mélange d'effluents gazeux. Il peut s'agir de tout air vicié issu d'un bâtiment ou de tout mélange d'air vicié issu d'un bâtiment. L'effluent gazeux peut être de l'air vicié par l'occupation humaine, par la circulation de véhicules dans le bâtiment, par exemple d'un parking ou de plusieurs parking, ou aux abords des bâtiments, par exemple tunnels, voieries souterraines, espaces sous dalles, par les productions industrielles, de l'air issus des moyens de chauffage du bâtiment, notamment au gasoil ou au gaz. Selon l'invention, effluent gazeux peut être un gaz comprenant par exemple du CO₂, du dioxyde d'azote ou de l'air viciés par l'occupation humaine, par la circulation de véhicules dans le bâtiment (parkings), par la circulation ferroviaire, ou aux abords des bâtiments (tunnels, voieries souterraines, espaces sous dalles), par les productions industrielles.

La présente invention permet notamment de retraiter les effluents gazeux pollués en CO₂ et dioxydes d'azote, monoxyde de carbone, et autres polluants qui sont évacués des bâtiments fabriqués par les humains, notamment ceux précités, notamment par les centrales de ventilations et autres exemples cités ci-dessous.

Selon l'invention, le traitement de l'effluent gazeux peut consister par exemple à éliminer, c'est-à-dire extraire, de l'effluent gazeux du CO₂ et/ou du NO₂. Avantageusement, les algues transforment le CO₂ et/ou le NO₂ en oxygène qui peut permettre, par exemple, de renouveler l'atmosphère du bâtiment ou être évacué. Il peut s'agir d'autres gaz que ceux précités, et il peut s'agir également de particules et poussières présentes dans ces gaz. Le traitement peut être fonction de l'algue et/ou du microorganisme choisie. De la même manière, l'algue et/ou le microorganisme peut être choisie en fonction de l'effluent à traiter.

Selon l'invention, le moyen d'injection de l'effluent dans la culture d'algues et/ou microorganisme peut être connecté à un moyen de récupération de l'effluent à traiter permettant de récupérer le ou les effluents issus d'un ou de plusieurs bâtiment(s) et/ou d'un ou plusieurs ouvrage(s) d'art souterrain(s), par exemple une infrastructure souterraine de circulation automobile, ferroviaire, par exemple un tunnel autoroutier, un tunnel de métro pour les injecter dans la culture d'algues. Avantageusement la présente invention permet de traiter l'air vicié ou le liquide vicié d'un bâtiment en y connectant le dispositif précité utilisé dans la présente invention

Selon l'invention, le moyen de récupération dudit effluent peut être choisi dans le groupe comprenant un ventilateur, une pompe aspirante, un circuit d'aération, un circuit de climatisation, un circuit de filtration d'air d'un bâtiment. Tout moyen de récupération d'un effluent à traiter peut être utilisé. Ce moyen de récupération peut être par exemple un tuyau (« pipeline ») permettant de transporter le ou les effluent(s) issu(s), par exemple, d'un bâtiment ou de plusieurs bâtiments, par exemple d'une ou plusieurs centrale(s) thermique(s) ou tout autre bâtiment tel que ceux précités. Bien entendu, ce moyen de récupération est connecté au moyen d'injection afin d'apporter le l'effluent au contact des algues et/ou des microorganismes en culture, lesdites algues et/ou des microorganismes ayant pour fonction de métaboliser les éléments polluants et/ou indésirables de l'effluent, par exemple gazeux, par exemple le CO₂ et/ou du NO₂ pour les éliminer.

Selon l'invention, le contenant de culture d'algue et/ou de microorganisme peut être tout contenant connu de l'homme du métier. Il peut se présenter par exemple sous une forme choisie dans le groupe comprenant un tube, un cylindre, un tube plat, un tube ondulé sur sa longueur et/ou sa largeur, un panneau creux, une sphère, un cube, un parallélépipède rectangle, une spirale, parallélépipède rectangle avec des bords arrondis, une forme creuse sans arrête vive, d'un sachet. Dans le vocabulaire dans le domaine de l'architecture, le terme « tube » couvre d'ailleurs toutes ces structures possibles, dès lors qu'elles sont creuses. De préférence, selon l'invention, la forme creuse est sans arrête vive. Aussi, dans la présente, par « tube », on entend toute forme de contenant permettant de contenir une culture d'algue et/ou de microorganisme, y compris un tube ou un panneau creux. Par exemple, il peut s'agir d'un sachet ultramince, par exemple un sachet en éthylène tétrafluoroéthylène (ETFE) ; d'un verre profilé, par exemple de forme parallélépipède, de préférence avec des bords arrondis, un panneau creux en verre profilé de préférence permettant une culture d'algues et/ou de microorganismes. Ce contenant forme en fait un réacteur dans lequel l'algue et/ou de microorganismes est cultivée. Ainsi, toute forme appropriée à la culture d'algue convient.

De préférence, la forme du contenant de culture d'algues et/ou de microorganismes est un parallélépipède rectangle creux avec des bords arrondis, par exemple un panneau creux avec des bords internes, et éventuellement externes, arrondis, ou un tube. Avantageusement, le dispositif de culture est sans arrête vive. En effet, l'absence d'arrête permet d'éviter l'accumulation et/ou l'accrochage des algues et/ou microorganismes qui est observée dans les contenants présentant des arrêtes vives, au niveau des creux formés par ces arrêtes.

Selon l'invention, le contenant est de préférence un contenant transparent à la lumière. Il peut s'agir par exemple d'un contenant en verre profilé, d'un tube en polycarbonate, en plexiglas. Il s'agit bien sûr de la paroi du contenant. Ceci est particulièrement préféré lorsque l'algue ou le microorganisme en culture nécessite pour croître et/ou traiter l'effluent de la lumière, et que la lumière utilisée est la lumière naturelle.

Selon l'invention, l'épaisseur du contenant, c'est-à-dire de sa paroi, peut être comprise entre 5 cm et 60 cm, de préférence entre 15 et 20 cm. Il peut s'agir en fait de toute épaisseur assurant la tenue du contenant lorsqu'il est rempli par le milieu de culture et les algues et ou microorganisme. L'homme du métier pourra aisément déterminer cette épaisseur.

Selon l'invention, la hauteur du contenant peut-être comprise par exemple entre 1 et 10 m, de préférence entre 2 et 8,50 m. En fait, tout hauteur peut être utilisée, pourvu qu'elle soit constructible.

Selon l'invention, lorsque le contenant est horizontal, la longueur du contenant peut être, par exemple égale à la longueur du bâtiment et/ou à sa largeur, par exemple 100 mètres.

Avantageusement, lorsque le contenant est horizontal, il peut être disposé par exemple en serpentin ou en spirale autour et/ou sur le bâtiment.

Lorsque le contenant est horizontal ou incliné, il peut être disposé sur le toit du bâtiment. L'inclinaison du toit peut commander l'inclinaison du contenant.

Selon l'invention, le contenant peut comprendre un renfoncement dans lequel peut être placé ou logé un éclairage et/ou rétro-éclairage. Par exemple, le contenant peut avoir une forme, en coupe transversale, de haricot, la lumière pouvant être logée dans le creux du haricot. Des creux multiples, ou ondulations, peuvent être prévues pour loger un éclairage artificiel permettant de fournir aux algues et/ou microorganismes cultivés la lumière nécessaire à leur croissance et/ou au traitement de l'effluent. Il peut s'agir également d'un éclairage artificiel.

Selon l'invention, lorsque le contenant de culture est un tube ou un panneau creux, le tube ou panneau creux est également, et pour les mêmes raisons que celles précitées, transparent à la lumière à laquelle est sensible l'algue et/ou le microorganisme pour sa culture. Cela permet avantageusement à l'algue et/ou au microorganisme de profiter de la lumière naturelle et/ou artificielle pour son métabolisme, en particulier la photosynthèse, notamment en vue de traiter l'effluent. Comme indiqué précédemment, il peut s'agir par exemple d'un tube ou panneau en verre, d'un tube ou panneau en polycarbonate ou en plexiglas, ou en tout autre matériau convenant à la mise en oeuvre de la présente invention.

Selon l'invention, lorsqu'il s'agit d'un tube, le diamètre extérieur du tube peut être compris par exemple entre 20 et 100 cm, de préférence entre 40 et 80 cm. L'épaisseur peut être par exemple celles précitées.

Selon l'invention, la hauteur des tubes peut-être comprise par exemple entre 1 et 10 m, de préférence entre 2 et 8,50 m. Les mêmes remarques que celles-ci-dessus aux contenant en général sont applicables pour la hauteur.

Selon l'invention, le contenant, par exemple le tube ou le panneau, peut être par exemple multicouche. Il peut comprendre par exemple, de l'extérieur vers l'intérieur, de manière concentrique, une couche extérieure, une couche médiane et une couche intérieure, et comprendre en outre un moyen d'éclairage ou rétroéclairage. Le rétroéclairage permet d'éclairer la culture, pour les raisons indiquées ci-dessus, par exemple lorsque l'environnement ne donne pas suffisamment de lumière ou que l'utilisateur souhaite stimuler la culture des algues et/ou microorganismes.

Par couche on entend l'espace crée entre 2 contenants, par exemple tubes ou panneau, concentriques, c'est-à-dire placés l'un dans l'autre l'axe des contenants, par exemple tubes ou panneaux, étant parallèle et laissant un espace entre les contenants, par exemple les tubes ou panneaux. Par concentrique on entend un ou plusieurs contenants, par exemple tube(s) ou panneau(x), placé(s) dans un ou plusieurs autre(s) tube(s).

La couche est donc délimitée par les parois des contenants, par exemple les tubes et/ou panneaux. Les contenants peuvent être identiques ou différents dans leur constitution, c'est-à-dire forme et matériaux utilisés. La distance entre les contenants concentriques créée un espace délimité par les parois des contenants. Cet espace est fonction du diamètre de chacun des contenants disposés concentriquement, de préférence dans le sens de la longeur.

De préférence, la surface du contenant de culture en contact avec le milieu de culture est une surface qui inhibe ou empêche toute adhésion, notamment des algues et/ou microorganismes, sur la surface. Il peut s'agir par exemple d'une surface préalablement traitée avec un; produit chimique anti-adhésion (« antifouling »).

Le contenant peut être par exemple un contenant vertical ou horizontal ou incliné. Par exemple, l'inclinaison du contenant peut être comprise entre 0 et 90°. De préférence, le contenant est un contenant vertical ou horizontal. Il peut s'agir, par exemple, de tubes ou panneaux creux ou toute autre forme précitée, verticaux ou non, par exemple, l'inclinaison du contenant, par exemple des tubes ou autre, peut être comprise entre la vertical ou l'horizontal par exemple comprise entre 0 et 90°.

Selon la présente invention, par éclairage favorable à la culture d'algues et/ou de microorganismes, on entend l'éclairage naturel, par exemple la lumière du jour et/ou ou l'éclairage artificiel, par exemple émis par un moyen d'éclairage permettant de reproduire la lumière du jour ou une longueur d'onde suffisante pour la culture de l'algue et/ou du microorganisme.

Selon l'invention, un moyen d'éclairage peut être un moyen indépendant des algues et/ou microorganismes en culture, venant en plus ou en remplacement de l'éclairage naturel par le soleil. Selon l'invention, le moyen d'éclairage peut être réalisé par exemple par un ou plusieurs tube(s) lumineux fluorescent(s), des diodes électroluminescentes (« LED »), par une ou plusieurs lampe(s) halogène(s). De préférence, l'éclairage peut être réalisé par un ou plusieurs tube(s) lumineux fluorescent(s), des diodes électroluminescentes, par une ou plusieurs lampe(s) halogène(s) dont les longueurs d'ondes lumineuses sont choisies entre 430 et 660 nm, de préférence égale à 430 nm ou 660 nm. L'éclairage peut être ornemental et/ou fonction de l'algue et/ou du microorganisme et de ses besoins pour sa croissance et / ou le traitement de l'effluent.

Le moyen d'éclairage peut être placée dans un espace créé par la disposition concentrique des contenants, par exemple des tubes et/ou du panneau creux. Il peut également être placé et/ou fixé sur une autre surface, par exemple une façade d'un bâtiment et/ou provenir du bâtiment lui-même. Par exemple lorsque l'on utilise deux contenants, par exemple tubes ou panneaux, disposés concentriquement, l'un étant externe et l'autre étant disposé à l'intérieur, le rétroéclairage peut être placé dans le contenant interne ou dans l'espace créé entre le contenant externe et le contenant interne, afin de protéger l'éclairage du milieu de culture.

Selon l'invention, les algues peuvent être choisies par exemple dans le groupe comprenant des Chlorophycées, Chlorella, Parietochloris incisa, des Diatomées Amphora sp., Nitzchia sp., Chaetoceros sp., des Chrysophycées. En fait, avantageusement, selon l'invention, tout type d'algue convient, dès lors qu'elle peut être cultivée et traiter un effluent au sens de la présente invention. Avantageusement, il peut s'agir d'une ou d'un mélange de micro-algues permettant de former du biodiesel.

Selon l'invention, le microorganisme peut être choisi parmi, par exemple, les bactéries, les levures, les champignons. Avantageusement, selon l'invention, tout type de microorganisme convient, dès lors qu'il peut être cultivée et traiter un effluent au sens de la présente invention. De préférence, le microorganisme est une bactérie. De préférence, la bactérie est une cyanobactérie. De préférence, la cyanobactérie est choisie dans le groupe comprenant *Spirulina platensis, Chroococcales Chamaesiphon, Chroococcales Gloeabacter, Chroococcales Synechococcus, Chroococcales Glocothece, Chroococcales Cyanothece, Chroococcales Gloecocapsa, Chroococcales Synechoexstis, Pleurocapsales Dermocarpa, Pleurocapsales Xenococccus, Pleurocapsales Dermocarpella, Pleurocapsales Myxosarcina, Pleurocapsales Chroococcidiopsis, Oscillatoriales Spirulina, Oscillatoriales Arthrospira, Oscillatoriales Oscillatoria, Oscillatoriales Lyngbya, Oscillatoriales Pseudanabaena, Oscillatoriales Starria, Oscillatoriales Crinalium, Oscillatoriales Microcoleus, Nostacales Anabaena, Nostocales Aphanizomenon, Nostocales Nodularia, Nostocales Cylindrospermum, Nostocales, Nostocales Scytonema, Nostocales Calothrix, Stigonematales Chlorogioecopsis, Stigonematales Fischerella, Stigonematales Stigonema, Stigonematales Geitteria, Plochloraceae Prochloron.*

La culture des algues et/ou des microorganismes peut être réalisée par tout moyen approprié connu de l'homme du métier. Selon l'Invention, Le milieu de culture peut être choisi en fonction de l'algue ou des algues pour permettre une culture de préférence optimale, mais surtout un métabolisme optimal pour le traitement de l'effluent, par exemple l'effluent gazeux. Les algues peuvent être stressées au cours de la culture pour augmenter leur efficacité de traitement de l'effluent gazeux ou liquide. Selon l'Invention, le milieu de culture peut être choisi en fonction du microorganisme ou des microorganismes pour permettre une culture de préférence optimale, mais surtout un métabolisme optimal pour le traitement de l'effluent, par exemple l'effluent gazeux. Les microorganismes peuvent être stressés au cours de la culture pour augmenter leur efficacité de traitement de l'effluent gazeux ou liquide.

De très nombreux milieux de culture sont accessibles sur internet et dans les ouvrages spécialisés. Selon l'invention, on privilégie les cultures en milieu aqueux. Le stress peut être apporté par exemple au moyen de molécules chimiques. L'homme du métier connaît ces techniques et molécules.

Le moyen d'alimentation de la culture d'algues et/ou de microorganismes peut comprendre par exemple une pompe automatique, des moyens de régulation de l'alimentation des algues, un réservoir d'alimentation. Tout autre moyen approprié pour assurer la culture de l'algue convient Tout ces moyens sont ceux classiquement utilisés par l'homme du métier pour assurer une culture continue d'algues ou de microorganismes.

Le moyen de régulation de la température de la culture d'algues et/ou de microorganismes peut être par exemple un thermostat ou tout autre moyen approprié de contrôle de la température et de réaction en cas de variation non désirée de la température.

Le dispositif utilisé dans de l'invention peut comprendre en outre un moyen de contrôle de la température autour du contenant de culture. Ces moyens de contrôle peuvent être connectés à des moyens de chauffage et/ou de refroidissement.

Selon l'invention, le moyen de chauffage de la culture d'algues et/ou de microorganismes peut être choisi par exemple dans le groupe comprenant un moyen de récupération de la chaleur d'un bâtiment, un moyen de récupération de la chaleur extérieure, un moyen de récupération de la chaleur solaire, un moyen de récupération de l'énergie calorifique.

Par exemple, le moyen de récupération de la chaleur extérieure peut être une pompe à chaleur. Le moyen de récupération de la chaleur bâtiment est une double peau comprenant une enveloppe extérieure disposée devant une façade d'un bâtiment.

Selon l'invention, le moyen de refroidissement de la culture d'algues et/ou de microorganismes peut être choisi par exemple dans le groupe comprenant un moyen de récupération de la fraicheur d'un bâtiment, un moyen de récupération de la fraicheur extérieure, un moyen de réfrigération, par exemple une climatisation. Par exemple, le moyen de récupération de la fraicheur extérieure peut être une pompe à chaleur. Le moyen de récupération de la fraicheur extérieur et/ou d'un bâtiment est une double peau comprenant une enveloppe extérieure disposée devant une façade d'un bâtiment.

Selon l'invention, le moyen de refroidissement et de chauffage peut être identique, par exemple, il peut s'agir de la double peau.

Selon l'invention, l'enveloppe extérieure de la double peau ou double paroi peut être transparente et peut comprendre des volets d'aérations, ladite double peau étant de préférence fixe et parallèle à une façade du bâtiment. L'enveloppe extérieure de la double peau peut être par exemple une surface vitrée, une surface en ETFE, une surface constituée de menuiserie et vitrage, de menuiserie et de résilles en acier, en métal, en inox, en galva, en nylon ou carbone. Le contenant de culture d'algues et/ou de microorganismes est positionné entre ladite façade du bâtiment du bâtiment et ladite enveloppe extérieure. Les volets d'aération permettent d'aérer l'espace entre l'enveloppe extérieure de la double peau ou double paroi et la façade du bâtiment.

Selon l'invention, l'enveloppe extérieure de la double peau est de préférence étanche, de préférence, elle peut permettre de limiter les passages d'air et/ou courants d'air venant de l'extérieur, notamment en hiver, afin de protéger le contenant de culture des algues des variations de température.

Selon l'invention, la double paroi ou la double peau permet avantageusement de former un matelas d'air autour du bâtiment et/ou de confiner de la chaleur et/ou la fraîcheur émise par le bâtiment, l'utilisation du dispositif pouvant ainsi permettre d'utiliser la chaleur et/ou la fraicheur émise par le bâtiment pour chauffer et/ou rafraichir la culture d'algues et/ou de microorganismes.

Avantageusement, la double peau permet en outre de stocker les calories du bâtiment, de d'améliorer l'inertie calorique d'un bâtiment et de réguler la température de la culture.

Avantageusement, la présente invention permet de récupérer la chaleur et/ou la fraîcheur d'un bâtiment pour cultiver les algues et/ou microorganisme et en même temps, grâce à la culture des algues et/ou microorganisme, de traiter des effluents générés, par exemple par ledit , bâtiment

Selon l'invention, le dispositif utilisé peut comprendre en outre un moyen de récupération de la biomasse formée par culture des algues.

Selon l'invention, ledit dispositif peut comprendre en outre un système de vidange du contenant de culture des algues et/ou microorganismes. Ce système peut permettre par exemple de nettoyer le contenant et/ou le dispositif de la présente invention en entier. Il permet également, si souhaité, de récupérer la biomasse formée pour la réutiliser comme indiqué ci-dessus.

Selon l'Invention, ledit dispositif utilisé peut comprendre en outre un ou plusieurs des moyens de contrôle et régulation de la culture des algues et/ou microorganismes suivants : moyens de contrôle de l'alimentation des algues, moyens de contrôle de l'injection de l'effluent à traiter, moyens de contrôle de la température, moyens de contrôle du pH, moyens de contrôle de l'éclairage des algues. Ces moyens peuvent être par exemple ceux couramment utilisés pour la culture d'algues et/ou de microorganismes, et de manière plus générale de microorganismes.

Avantageusement, ledit dispositif utilisé peut être piloté par ordinateur afin de permettre une optimisation de la culture des algues et/ou microorganismes et/ou du traitement de l'effluent L'ordinateur peut être connecté par exemple au thermostat du contenant de culture d'algues et/ou de microorganismes et aux différents moyens de contrôle installés pour faire fonctionner le dispositif de l'invention.

Le dispositif utilisé dans la présente invention peut fonctionner en continu ou en discontinu. Il peut fonctionner, sans interruption, jour et nuit L'éclairage des algues et/ou microorganismes peut ainsi être maintenu en permanence de manière à maintenir la culture active pour le traitement de l'effluent

La façade du bâtiment peut comprendre, avantageusement, un garde-corps. Le garde corps peut permettre, par exemple la circulation d'individus le long du dispositif, l'entretien et la maintenance du dispositif.

Par exemple, le garde corps peut, par exemple, être constitué de métal, de verre. Par exemple, il peut être constitué de menuiseries en métal et résille en acier, en métal, en inox, en galva, en nylon ou carbone, de barreaux en métal et/ou de menuiserie en métal et de membrane ETFE.

Selon l'invention, le dispositif utilisé peut comprendre en outre un renfort de structure permettant de supporter le contenant de culture d'algues et/ou de microorganismes, le contenant de culture d'algues et/ou de microorganismes pouvant être par exemple un tube de culture d'algues et/ou de microorganismes, ledit renfort pouvant être fixé au bâtiment. Ce renfort de structure peut être fixé sur le bâtiment, être autosupporté ou être fixé sur l'enveloppe extérieure de la double peau. Lorsque le contenant de culture des algues est disposé sur une façade d'immeuble, par exemple, cela permet de le supporter.

Selon l'invention, ledit dispositif utilisé peut comprendre en outre au moins une coursive, ladite coursive pouvant servir de support du contenant de culture d'algues et/ou de microorganismes et/ou de moyen d'accès au contenant de culture d'algues et/ou de microorganismes pour du personnel d'entretien.

Ainsi, selon l'invention, avantageusement, on peut former une véritable biofaçade d'un bâtiment, biofaçade qui permet non seulement de traiter l'air vicié émis dans et par le bâtiment, d'isoler le bâtiment, de récupérer la chaleur du bâtiment pour une culture d'algues et/ou de microorganismes, et permet en outre de fabriquer une biomasse réutilisable notamment en tant que biocarburant, dans le domaine de la pharmacie, dans le domaine agroalimentaire. Il peut d'agir par exemple de composés chimique, de protéines.

Selon l'invention, avantageusement la biomasse qui peut être formée par la culture d'algues et/ou de microorganismes peut en effet être récupérée et réutilisée, notamment comme biocarburant, comme composé chimique, composé pharmaceutique.

La biomasse peut permettre de fabriquer un microorganisme oléagineux.

Selon l'invention, la biomasse peut être une biomasse transformable en charbon ou en biopétrole, par exemple par les techniques connues de l'homme du métier.

Selon l'invention, la biomasse peut être utilisée directement pour produire de l'électricité. Elle peut également être acheminée, par exemple par un tuyau à un bâtiment, par exemple une centrale de traitement. Elle peut également être transformée, par exemple par pressage en huile ou traitement par pyrolyse par les techniques connues de l'homme du métier pour produire du charbon ou du biopétrole.

Selon l'invention, avantageusement, le dispositif peut être intégré à la structure d'un bâtiment ou rapporté sur le bâtiment.

La présente invention permet ainsi de faire produire aux façades des bâtiments de l'énergie primaire biochimique obtenue par photosynthèse notamment de biocarburants.

Avantageusement, la présente invention permet d'utiliser les opportunités climatiques, chimiques et structurelles qu'offrent les façades des bâtiments, pour intégrer un procédé biochimique à l'intérieur ou le long des façades, de travailler sur un état de symbiose entre les deux systèmes qui se servent mutuellement, l'un recyclant les émanations de l'autre pour produire l'énergie dont il a besoin.

La présente invention peut avantageusement être mise en oeuvre sur toutes les surfaces de bâtiments, enveloppes de bâtiments, façades ou toitures, ouvrage d'art souterrain, par exemple une infrastructure souterraine de circulation automobile, ferroviaire, par exemple un tunnel autoroutier, un tunnel de métro, neufs ou existant. Par exemple, la surface peut être une partie faiblement exploitée des bâtiments par exemple l'enveloppe extérieure ou toute autre surface ou toutes les surfaces du bâtiment ou une surface intérieur du bâtiment. La surface choisie pour placer le dispositif de l'invention est préférentiellement une surface qui permet de profiter d'une grande surface développée disponible, d'une disposition en hauteur, d'un rayonnement incident, d'émanations thermiques, de son infrastructure, et des apports chimiques du bâtiment. La surface peut être, par exemple, une surface en béton, une surface vitrée, une surface constituée par un complexe étanche et un bardage, une surface photovoltaïque. Les conditions optimales sont celles de la culture des algues choisies.

En limitant l'emprise foncière au sol du dispositif de l'invention, cette invention peut trouver sa place n'importe où, même en milieu urbain déjà densifié. Elle peut en outre être appliquée à des façades d'immeubles existants lors d'opérations de réhabilitation ou restructurations. En cela elle répond aux campagnes de restructurations actuelles d'immeubles de bureaux pour les mettre aux nouvelles exigences et normes environnementales

Du point de vue du bâtiment, la présente invention peut avantageusement intégrer les évolutions récentes des constructions bioclimatiques. Par rapport aux façades énergétiques traditionnelles de type photovoltaïques, elle a l'avantage de pouvoir fonctionner sur n'importe quelle orientation.

Le contenant de culture des algues et/ou de microorganisme peut être un bioréacteur. Il peut être disposé sur une surface telle que définie précédemment. Le bioréacteur peut, par exemple être sur et/ou devant une façade d'un bâtiment et peut être desservi par des coursives extérieures.
- les coursives permettent avantageusement de protéger la culture d'algues et/ou de microorganismes des surchauffes solaires en été
- les bioréacteurs verticaux ou horizontaux réalisent une protection solaire complémentaire
- une limitation des surfaces vitrées de l'enveloppe extérieure de la double peau permet avantageusement d'éviter les surchauffes d'été et est en outre compatible avec la surface disponible pour l'éclairement des locaux et laissée libre par le dispositif
- l'inertie du bâtiment peut encore être améliorée par des tubes et/ou panneaux creux contenant de l'eau en façade qui régulent ses surchauffes thermiques. Ces tubes d'eau et/ou panneaux creux peuvent permettre de réguler la température de culture des algues et/ou microorganismes, mais peuvent également participer à alimenter la culture d'algues et/ou de microorganismes.

Le contenant de culture lui-même peut être considéré comme un contenant d'eau et permet donc également de participer à la régulation des surchauffes thermiques.

Le dispositif utilise dans de la présente invention peut comprendre en outre, par exemple, l'incorporation de moyens permettant une amélioration de la protection thermique des bioréacteurs.

Le contenant de cultures d'algues et/ou de microorganismes ou bioréacteur, tel que défini précédemment peut être intégré à une façade double peau ventilée. Cette double façade est la double peau définie ci-dessous.

Le contenant de culture est compris entre deux surface « surface intérieure », par exemple une surface de bâtiment, et une surface extérieure, c'est à dire une surface autre que la surface intérieure, par exemple l'enveloppe extérieure, ces deux surfaces définissant une double peau.

Les avantages de la double peau sont les suivants :
- les mêmes que ceux de l'invention, avec en plus:
- Un effet de serre avec apports caloriques gratuit en hiver
- Un espace rafraîchi et ventilé en été par ouverture de la double peau
- Une récupération directe des énergies dissipées par l'enveloppe du bâtiment qui sont récupérée par le procédé.
- Une utilisation optimisée de l'inertie thermique des tubes d'eau qui permet de réguler cet espace intermédiaire en stockant la chaleur excédentaire et en la restituant lorsque les apports énergétiques solaires diminuent

L'enveloppe extérieure de la double peau ou surface extérieure peut être composées par exemple de :
- vitrages fixes, ouvrants ou coulissants
- de jalousies vitrées horizontales, verticales ou obliques laissant passer la lumière
- les surfaces gonflables en éthylène tétrafluoroéthylène (ETFE) avec ventilation intégrée
- de surfaces composites ou tout autre matériau ou système assurant une transparence et/ou une ventilation suffisante aux besoins du bâtiment

La double paroi ou double peau permet avantageusement de réaliser une façade pariétodynamique permettant de récupérer les énergies ou calories excédentaires pour les retransformer en froid et/ou en chaleur, par exemple par l'usage d'une PAC (pompe à chaleur). En hiver, le système peut s'inverser avec une PAC inversable.

Le contenant de culture des algues et/ou microorganismes disposé à l'intérieur d'un bâtiment est également décrit.

Le bâtiment peut être par exemple, un bâtiment étudié pour minimiser ses dépenses énergétiques et ses flux.

Le concept de mutualisation peut être global et englobe l'ensemble du bâtiment.

Par exemple des ventilations de façade à façade peuvent permettre un prétraitement ou un recyclage thermique de l'air en utilisant l'espace de la double façade et l'inertie donnée par les masses d'eau. Avantageusement, par exemple en été la double façade ou double peau peut être une façade qui est ouverte à plus de 50% : l'ombrage donné par les installations et les coursives peut limiter les apports solaires et donc la climatisation. En hiver, la double façade peut être une double peau fermée qui peut permettre la formation d'un matelas d'air isolant autour du bâtiment.

Un des nombreux objectifs atteints par la présente invention est d'obtenir une construction:
- la plus passive possible, d'où l'intérêt du stockage d'eau, de l'effet protection solaire et de l'espace isolant de la double façade
- autonome énergétiquement voir sur-productive.

La présente invention permet, par exemple, de
- dépolluer, car le bioréacteur permet, par exemple de transformer du gaz carbonique et du dioxyde d'azote en oxygène, d'éliminer, par exemple du plomb, du nickel, par exemple des nitrates et/ou à désaliniser un effluent liquide.
- produire des microorganismes oléagineux utilisables pour produire de l'énergie, de la matière organique, par exemple des protéines, des composés chimiques.

La production énergétique de la biomasse ainsi produite par la présente invention est 130 fois plus performante à surface de culture égale qu'une production d'agrocarburant équivalente issue de l'agriculture traditionnelle, les récoltes se faisant sur un cycle court de 1 à 3 jours pour plusieurs mois en agriculture de plein champ.

La présente invention permet également de produire toutes sortes de sous produit intéressants, par exemple des protéines, des composants, de la matière organique utilisable, par exemple dans le domaine pharmaceutique.

La présente invention permet, en outre de produire la matière première énergétique à l'endroit même où elle va être consommée et donc:
- d'éviter toute perte de charge liée au transport
- un recyclage en boucle des déchets de combustion.

Elle permet, également de recycler intégralement les émanations de CO₂ liés à la production énergétique.

Elle permet, en plus de produire de l'énergie à partir de déchets de combustion jusqu'à lors dilapidés dans l'atmosphère

La présente invention permet, par exemple, d'optimiser la production et le rendement des bioréacteurs connus de l'état de la technique, par exemple les bio-réacteurs de la société Biopetroleo BASF en Espagne ceux de la société Samash en France, de la société Greenfuel , de la société Shell/Petrosun et de la société Valcent aux USA, de la société Alguatech en Israel, de la société Aquafl ow bionomic corporation en Nouvelle Zélande en mettant à disposition de celui-ci des surfaces de façades, et une infrastructure, et, dans le cas des doubles façades, un espace tempéré, prérafraichi ou préchauffé gratuitement par la double façades et le recyclage des déperditions thermiques et émanations d'air du bâtiment.

La présente invention contribue également à la régulation thermique du bâtiment en autorisant des doubles façades à vocation agricole (non prises en compte dans la surface hors oeuvre nette (SHON), là où le règlement définissant la SHON interdit de réaliser des doubles façades classiques car elles seraient alors prises en compte dans le calcul de la SHON et feraient donc baisser de façon rédhibitoire la performance foncière du rapport SHON/SUB

Elle contribue aussi à la régulation thermique du bâtiment en introduisant dans les doubles façades des masses d'eau qui créent une forte inertie à même de « lisser » les pics énergétiques découlant des aléas climatiques ou liés au rythme nycthéméral du bâtiment.

### Brève description des figures :

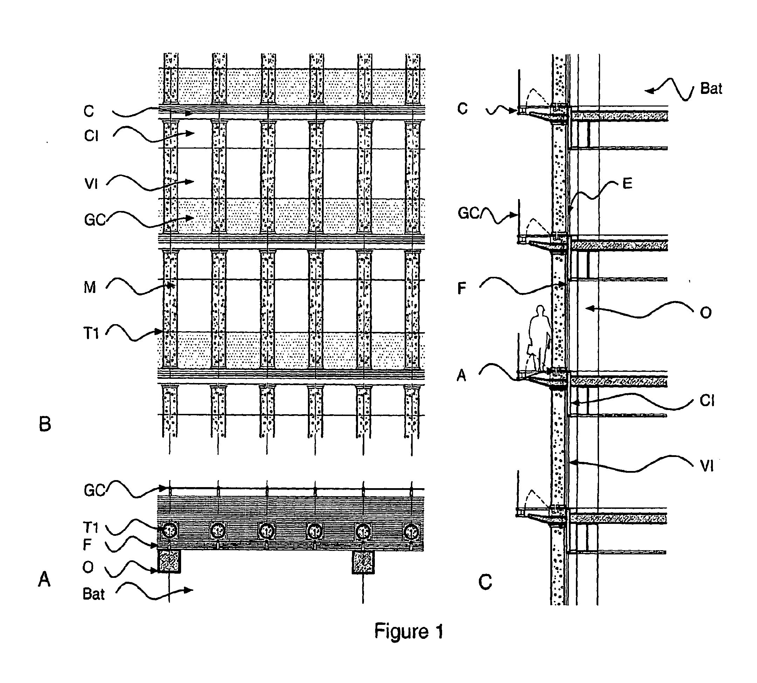

- La figure 1 représente un mode de réalisation au dehors de l'invention. La figure A représente une vue de dessus du dispositif de l'invention, en particulier une façade (F), les tubes T1 disposés entre la façade du bâtiment (Bat) et un garde-corps (GC). Les tubes comprennent un milieu de culture (M). La figure 1 B une vue de face de la façade (F) et des garde-corps (GC) fixés sur la coursive (C) par boulonnage. La figure C représente une coupe transversale du bâtiment sur laquelle est indiquée la façade du bâtiment (F) avec une alternance de panneaux de verre (VI) et de complexe isolant (CI), la coursive (C), les tubes T1 sont fixés sur la coursive (C) à l'ossature primaire(O) par l'intermédiaire d'une cornière (A).

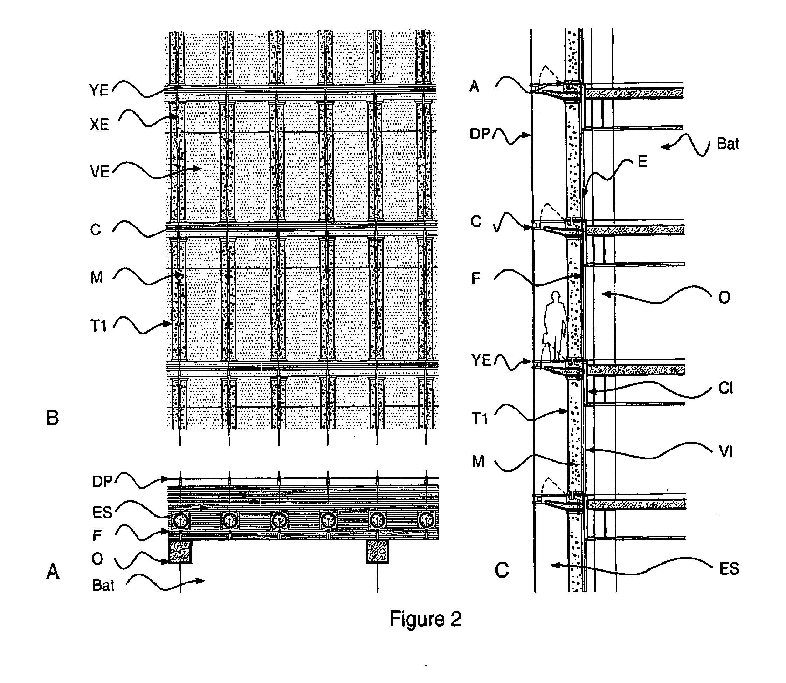

- La figure 2 représente un mode de réalisation de l'invention. La figure 2A représente une vue de dessus du dispositif de l'invention, en particulier une façade (F), les tubes (T1) disposés entre la façade du bâtiment (Bat) et l'enveloppe extérieure de la double peau (DP). Les tubes comprennent un milieu de culture (M). La figure 2B une vue de face de la façade (F) et des panneaux de verre (VE) de l'enveloppe extérieure de la double peau (DP) soutenue par des montants extérieurs (XE) et des traverses extérieures (YE). La figure 2C représente une coupe transversale du bâtiment sur laquelle est indiquée la façade du bâtiment (F) avec une alternance de panneaux de verre (VI) et de complexe isolant (CI), la coursive (C), les tubes (T1) sont fixés sur une coursive (C) à la structure primaire (O) par l'intermédiaire d'une cornière (A). Les tubes sont positionnés derrière la double peau (DP).

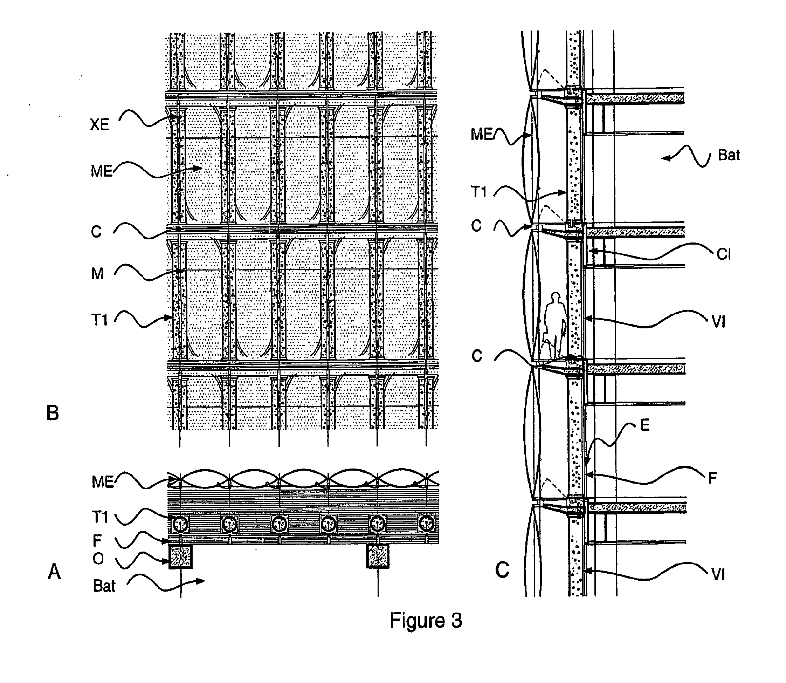

- La figure 3 représente un mode de réalisation de l'invention. La figure 3A représente une vue de dessus du dispositif de l'invention, en particulier une façade (F), les tubes T1 disposés entre la façade du bâtiment (Bat) et l'enveloppe extérieure constituée d'une membrane en éthylène tétrafluoroéthylène. Les tubes comprennent un milieu de culture (M). La figure 3 B une vue de face de la façade (F) et de la membrane en éthylène tétrafluoroéthylène (ME) soutenue par des montants extérieurs (XE). La figure 3C représente une coupe transversale du bâtiment sur laquelle est indiquée la façade du bâtiment (F) avec une alternance de panneaux de verre (VI) et de complexe isolant (CI), la coursive (C), les tubes (T1) sont fixés sur la coursive (C) à la structure primaire(O) par l'intermédiaire d'une cornière.

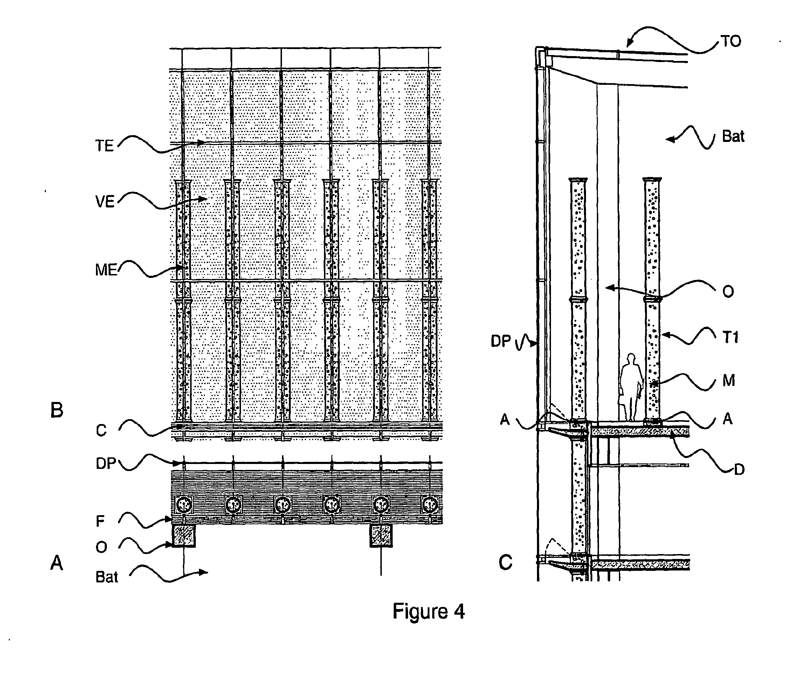

- La figure 4 représente un mode de réalisation de l'invention. La figure A représente une vue de dessus du dispositif de l'invention, en particulier une façade (F), les tubes T1 disposés entre la façade du bâtiment (Bat) et l'enveloppe extérieure de la double peau (DP) et à l'intérieur du bâtiment. Les tubes comprennent un milieu de culture (M). La figure 4 B une vue de face de la façade (F) et des panneaux de verre (VE) de l'enveloppe extérieure de la double peau (DP) soutenue par des montants extérieurs (XE) et des traverses (TE). La figure C représente une coupe transversale du bâtiment sur laquelle est indiquée la façade du bâtiment (F) et la toiture. (TO) fixé au bâtiment (Bat) par l'intermédiaire de la structure primaire (O), les tubes (T1) sont fixés sur le plancher (D) par l'intermédiaire d'une cornière (A). Les tubes sont positionnés derrière la double peau (DP) et sous la toiture (TO) et à l'intérieur du bâtiment (Bat).

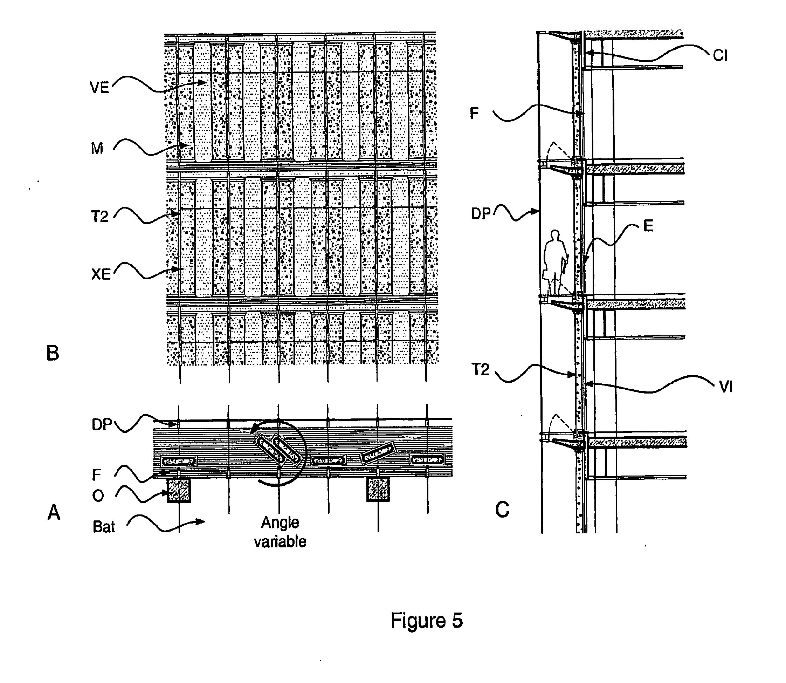

- La figure 5 représente un mode de réalisation de l'invention. La figure 5A représente une vue de dessus du dispositif de l'invention, en particulier une façade (F), les panneaux creux (T2) disposés entre la façade du bâtiment (Bat) et l'enveloppe extérieure de la double peau (DP). Les panneaux comprennent un milieu de culture (M). La figure 5 B une vue de face de la façade (F) et des panneaux de verre (VE) de l'enveloppe extérieure de la double peau (DP) soutenue par des montants extérieurs (XE). La figure 5C représente une coupe transversale du bâtiment sur laquelle est indiquée la façade du bâtiment (F) avec une alternance de panneaux de verre (VI) et de complexe isolant (CI), la coursive, les panneau creux T2 sont fixés sur la coursive à la structure primaire(O) par l'intermédiaire d'une cornière. Les panneaux creux sont positionnés derrière la double peau (DP) selon différents angles.

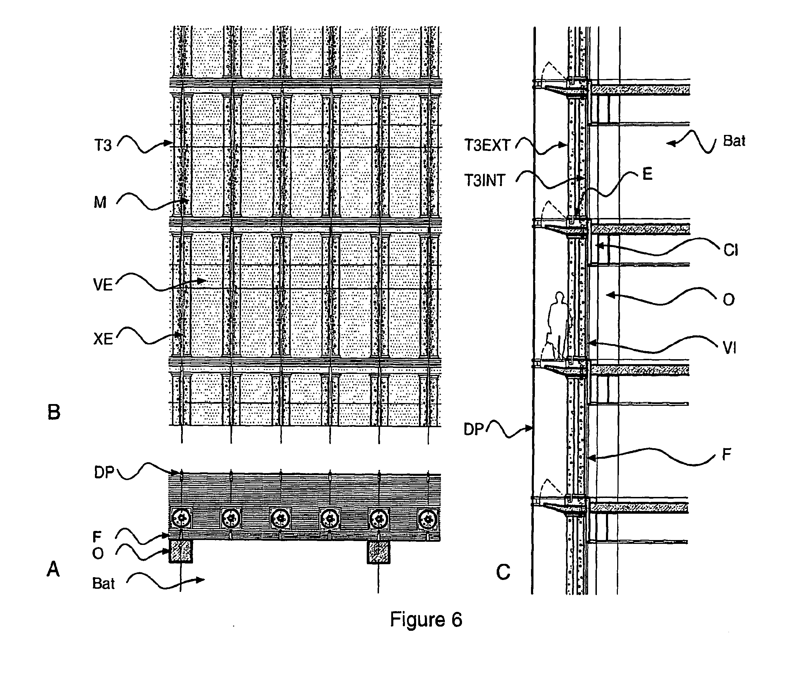

- La figure 6 représente un mode de réalisation de l'invention. La figure 6A représente une vue de dessus du dispositif de l'invention, en particulier une façade (F), les tubes (T3) sont disposés entre la façade du bâtiment (Bat) et l'enveloppe extérieure de la double peau (DP). Les tubes comprennent un milieu de culture (M). La figure 6 B une vue de face de la façade (F) et des panneaux de verre (VE) de l'enveloppe extérieure de la double peau (DP) soutenue par des montants extérieurs (XE). La figure 6C représente une coupe transversale du bâtiment sur laquelle est indiquée la façade du bâtiment (F) avec une alternance de panneaux de verre (VI) et de complexe isolant (CI), la coursive (C), les tubes T3 sont fixés sur la coursive (C) à la structure primaire (O) par l'intermédiaire d'une cornière. Les tubes sont positionnés derrière la double peau (DP). Ils sont constitués d'un tube extérieur (T3ext) et un tube intérieur (T3int)

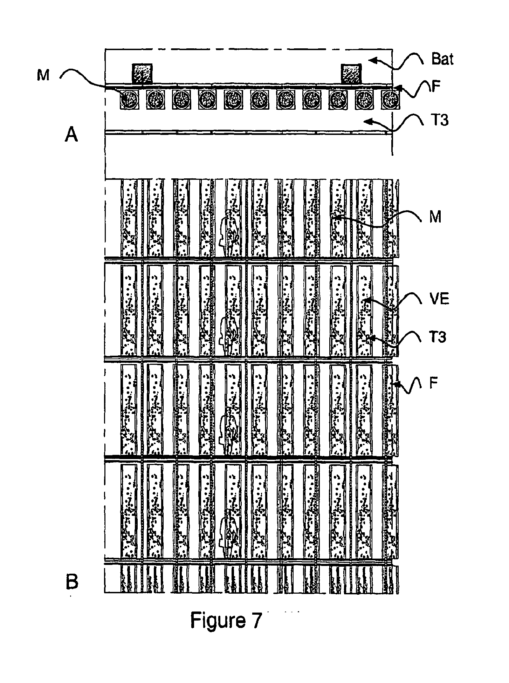

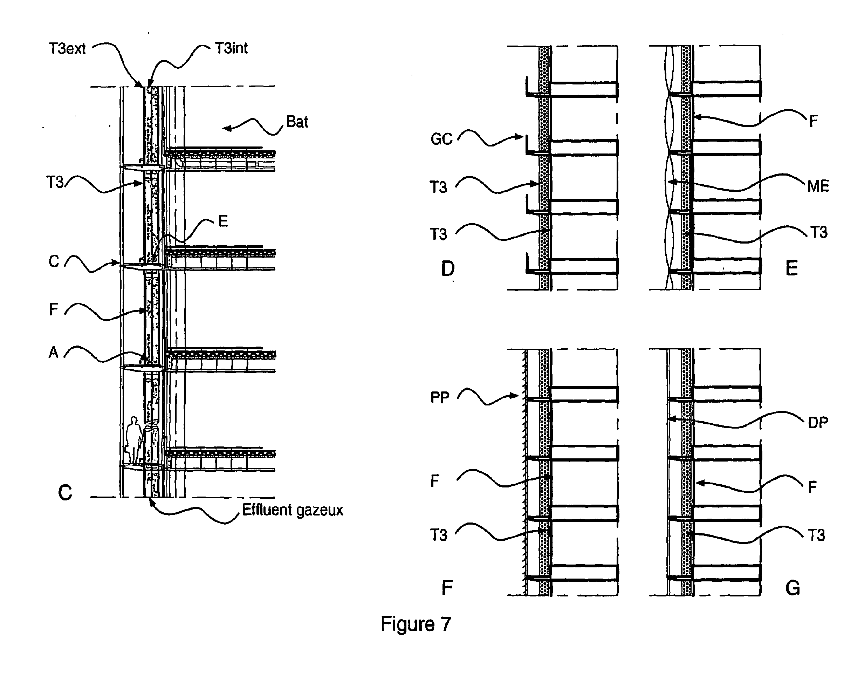

- La figure 7A représente une vue de dessus du dispositif, elle est identique à la figure 1 A. La figure 7 B représente une vue de face, elle est identique à la figure 1 B. La figure 7 C représente une coupe transversale du bâtiment, elle est identique à la figure 1 C. La figure 7 D, hors de l'invention, représente un tube T3 positionné entre la façade F et un garde-corps (GC). La figure 7 E représente un mode de réalisation de l'invention un tube T3 positionné entre la façade F et une membrane en éthylène tétrafluoroéthylène (ME). La figure 7 F représente un mode de réalisation de l'invention un tube T3 positionné entre la façade F et une enveloppe extérieure (PP) constituée de persiennes en verre. La figure 7 G représente un mode de réalisation de l'invention un tube T3 positionné entre la façade F et une enveloppe extérieure (DP).

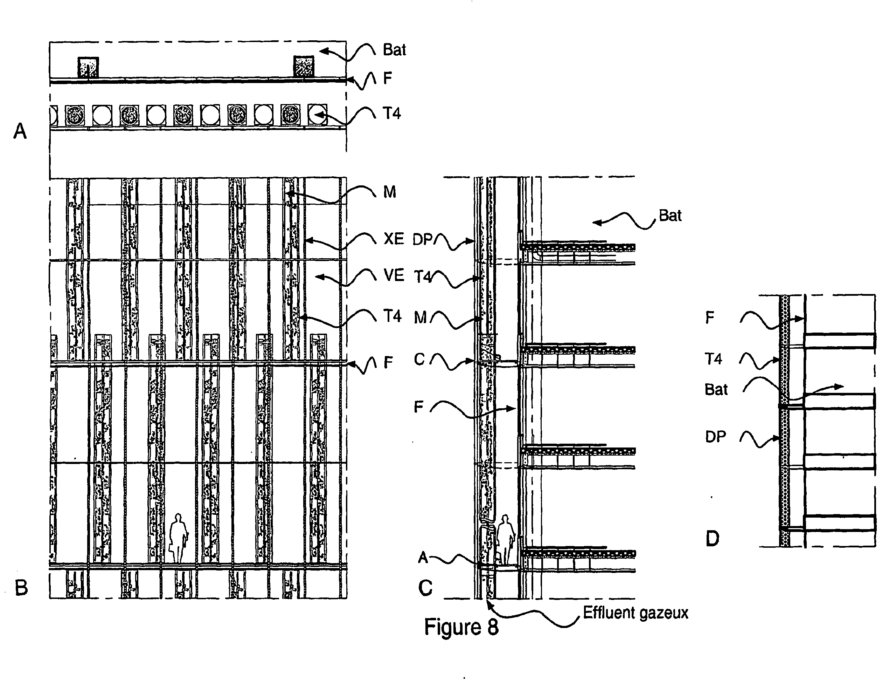

- La figure 8 représente un mode de réalisation de l'invention. La figure 8A représente une vue de dessus du dispositif de l'invention, en particulier une façade (F), les tubes (T4) disposés entre la façade du bâtiment (Bat) et l'enveloppe extérieure de la double peau (DP). Les tubes comprennent un milieu de culture (M) et sont positionnés contre la double peau (DP). La figure 8 B une vue de face de la façade (F) et des panneaux de verre (VE) de l'enveloppe extérieure de la double peau (DP) soutenue par des montants extérieurs (XE). La figure 8C représente une coupe transversale du bâtiment sur laquelle est indiquée la façade du bâtiment (F), la coursive (C), les tubes T4 sont fixés sur une coursive (C) à la structure primaire(O) par l'intermédiaire d'une cornière (A).Les tubes sont positionnés derrière la double peau (DP). La figure 8 D représente un tube (T4) positionné entre la double peau (DP) et la façade (F) du bâtiment.

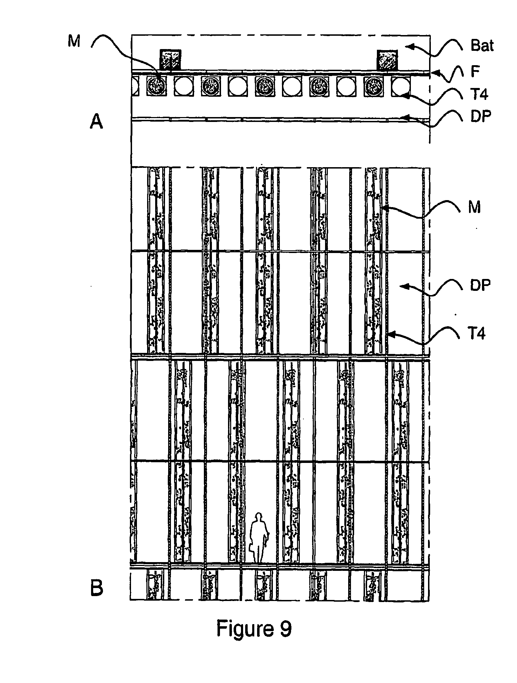

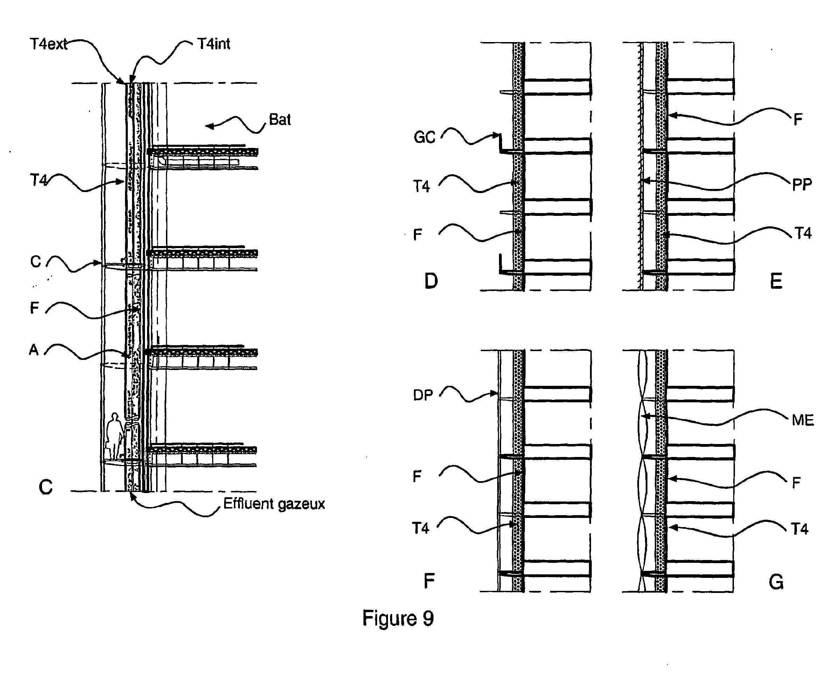

- La figure 9 représente un mode de réalisation de l'invention. La figure 9 A représente une vue de dessus du dispositif de l'invention, en particulier une façade (F), les tubes (T4) disposés entre la façade du bâtiment (Bat) et l'enveloppe extérieure de la double peau (DP). Les tubes comprennent un milieu de culture (M) et sont positionnés contre la façade (F). La figure 9 B est identique à la figure 8 B. La figure 9C représente une coupe transversale du bâtiment sur laquelle est indiquée la façade du bâtiment (F), les tubes (T4) sont fixés sur une coursive (C) à la structure primaire (O) par l'intermédiaire d'une cornière (A). Les tubes sont positionnés derrière la double peau (DP). La figure 9 D représente un tube (T4) positionné entre la façade (F) et un garde-corps (GC). La figure 9 G représente un tube (T4) positionné entre la façade (F) et une membrane en éthylène tétrafluoroéthylène (ME). La figure 9 E représente un tube (T4) positionné entre la façade (F) et une enveloppe extérieure (PP) constituée de persiennes en verre. La figure 9 F représente un tube T4 positionné entre la façade (F) et une enveloppe extérieure (DP).

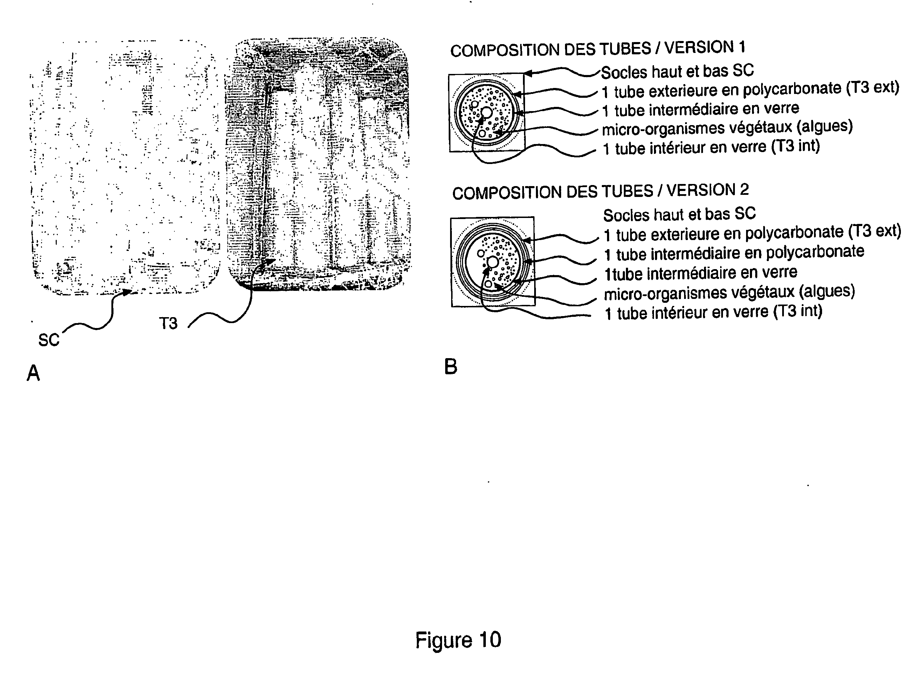

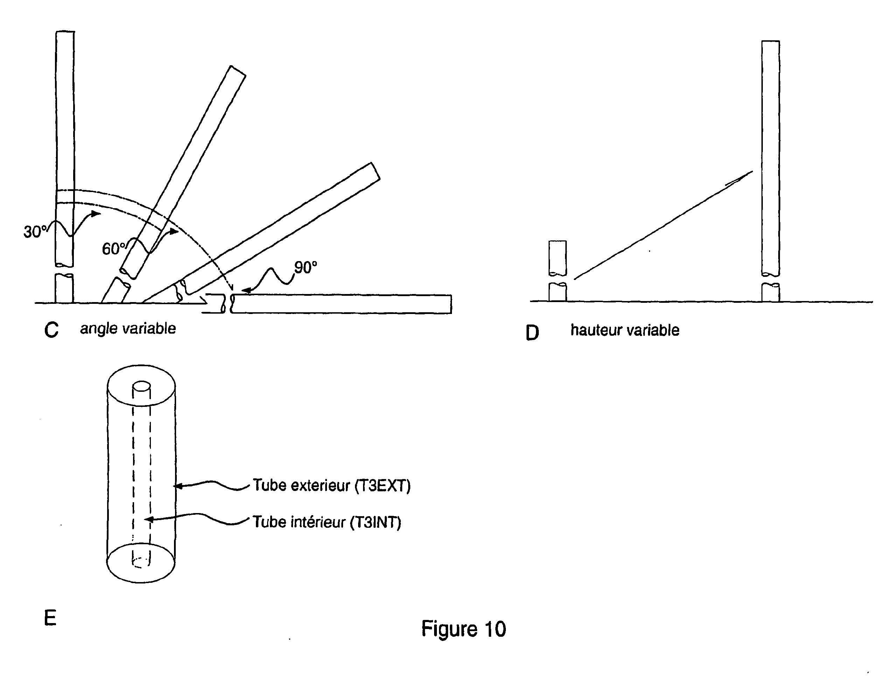

- La figure 10 A est une photographie représentant des tubes verticaux de cultures d'algues (T3) avec un support de culture (SC). La figure 10 B représente deux coupes transversales de deux tubes multicouches avec un tube intérieur (Tint), un tube intermédiaire (Ti) et un tube extérieur (Text). La figure 10 C représente différents modes de réalisation du dispositif à savoir différentes inclinaisons et la figure 10 D représente lesdifférentes hauteurs des tubes et la figure 10 E représente une vue en perspective d'un tube (T3) comprenant un tube intérieur (T3int) et un tube extérieur (T3ext) concentriques.

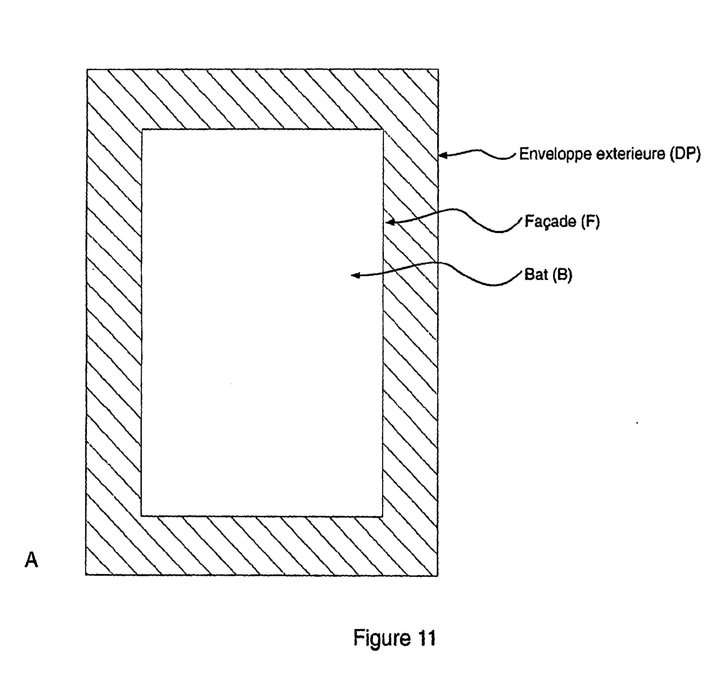

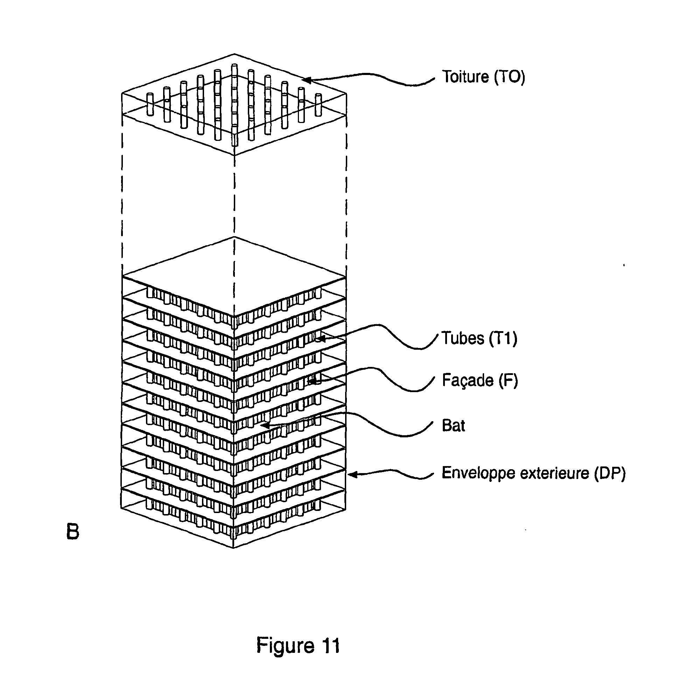

- La figure 11A est une représentation schématique d'un bâtiment (Bat) vue de dessus avec sa façade (F) et représentant l'enveloppe extérieure de la double peau (DP). La figure 11 B représente l'axonométrie éclatée d'un bâtiment avec des tubes (T1) fixés devant la façade (F) et derrière l'enveloppe extérieure de la double peau (DP). Le bâtiment est équipé de tubes (T1) verticaux supportés par la toiture et installés sous une toiture (TO)

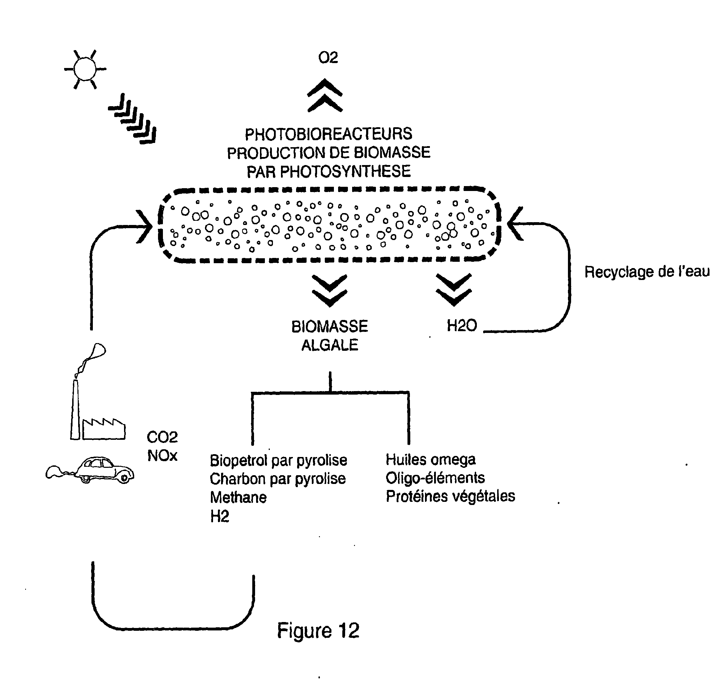

- La figure 12 est un schéma général représentant un exemple de traitement d'effluents gazeux avec un dispositif utilisé dans la présente invention.

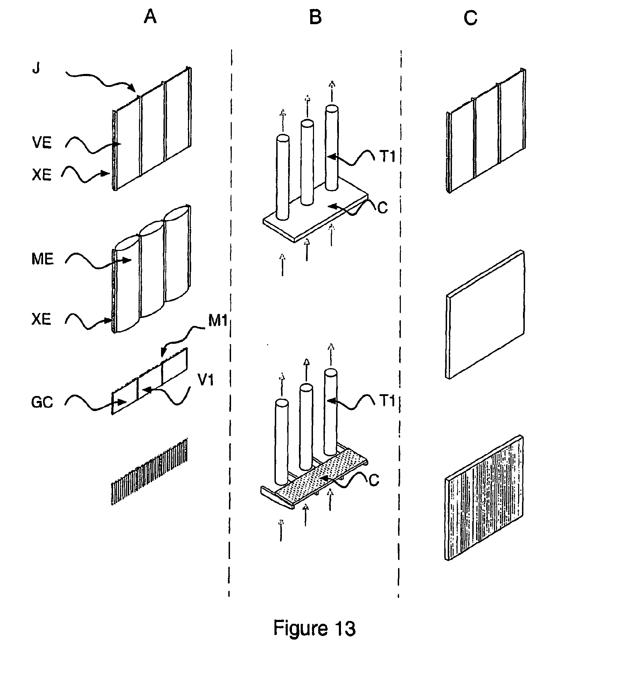

- La figure 13 représente différents exemples de réalisation de l'invention. La figure13 A représente différentes doubles façades et garde corps à savoir une dans lequel la double façade est une double façade en verre (VE) avec des montants métalliques (XE) et des joints étanches (J), une double façade avec des montants métalliques (XE) et une membrane ETFE (ME). Un garde corps en verre (V1) avec des montants métalliques (M1) et des joints étanches (J), un garde corps formé par des barreaux en métal.
- La figure 13 B représente différentes coursives en béton ou métallique. La figure 13 C représente différentes façades de bâtiments à savoir une façade constituée de menuiserie et de verre, un mur en béton ou un complexe étanche et bardage.

D'autres avantages pourront encore apparaître à l'homme du métier à la lumière des figures annexées, donnés à titre illustratif.

### EXEMPLES

### Exemple 1 : Dispositif hors de l'invention disposé sur une façade d'un bâtiment

On décrit ici un mode de réalisation de l'invention. Le dispositif utilisé est schématisé sur la figure 1.

La culture d'algue utilisée est Chlorella. Elle est cultivé dans un milieu aqueux de culture, à savoir le milieu de Walnes c'est-à-dire pour 10 litres de milieu 680g de nitrate de sodium (NaNO₃), 200g de Dihydrogénophosphate de sodium, 400g d'acide Ethylènediamine tetraacetique de sodium (Na₂EDTA), 20g d'acide borique (H₃BO₃). 40 ml d'une solution de 500 ml contenant 32,5 g de Bromure de potassium (KBr), 6,5g de chlorure de strontium (SrCl₂, 6H₂0), 0.25g de solution de chlorure d'aluminium (AlCl₃, 6 H₂O), 0,1g de chlorure de rubidium (RbCl), 0,05 g de chlorure de lithium (LiCl, H₂0), 0,025g de iodure de potassium (KI) et 800 mL d'une solution de 10 litres contenant 213,2 g de chlorure de fer hexahydraté (FeCl3, 6H₂0), 15,0 g de sulfate de manganèse monohydraté (MnSO4, H₂O), 2,5 g de sulfate de zinc (ZnSO4), 2,0 g de sulfate de cuivre pentahydraté (CuSO4, 5H₂0), 0,26 g de sulfate de cobalt heptahydraté (CoSO4, 7H₂0), 0,14 g de molybdate de sodium dihydraté (Na2Mo4, 2H₂0) et 0,10g de fluorure de sodium (NaF).

La culture de l'algue est effectuée dans le milieu précédent à une température de 20°C. Le pH du milieu est égal à 7,3.

Le moyen d'alimentation de la culture d'algue est la plaque elle-même qui permet à la fois de cultiver l'algue et de l'alimenter. Elle comprend en outre un moyen d'injection de l'effluent gazeux, qui est constitué par un orifice situé dans la pièce moulée, formant la base du tube (T1), d'un diamètre de 2 cm sur lequel est vissé un tuyau permettant l'acheminement de l'effluent gazeux dans la culture d'algue.

Le tube comprend également à sa base un deuxième orifice d'un diamètre de 7 cm sur lequel est vissé via un tube souple en polyéthylène d'un diamètre de 5 cm à une pompe d'alimentation LAMBDA PRECIFLOW (marque déposée).

La plaque comprend également un troisième orifice d'un diamètre de 7 cm situé dans la base moulée de la plaque connectée à une pompe LAMBDA PRECIFLOW (marque déposée) permettant l'aspiration du milieu de culture.

La température de la culture d'algue est contrôlée via une sonde de température commercialisée par la société Testo positionnée dans la base moulée de la plaque et plongée dans le milieu de culture.

Une sonde pHmètre de la société Testo est plongée dans le milieu de culture permettant ainsi le control du pH.

Une sonde de température de la société prosensor est plongée dans le milieu de culture.

Une sonde de mesure de la concentration du CO₂ dans le milieu est présente dans le milieu de culture.

Ces sondes sont connectées à un dispositif de contrôle permettant de suivre l'évolution du pH, de la concentration du CO₂ et de la température du milieu.

Tous les 24 heures, 50% du volume du milieu de culture est pompé et stocké dans un réservoir, le milieu pompé comprend des algues et correspond à de la biomasse. Un volume identique de milieu frais est simultanément injecté dans le contenant de culture. Le milieu de culture est ainsi renouvelé de manière à assurer une croissance optimale des algues et donc un traitement optimum des effluents.

Le contenant de culture est un tube (T1) représenté sur la figure 1. Le tube (T1) est constitué d'un verre profilé de forme cylindrique.

Le verre à une épaisseur de 5 cm, la hauteur du tube est de 3.2 M de haut. Le diamètre du tube est de 40cm. Elle comprend une pièce rectangulaire moulée de 50cm de large et de 50cm de long. Cette pièce fait tenir le verre par cerclage avec un la pièce moulée en partie basse et en partie haute, c'est-à-dire à une hauteur de 3 cm Et de 320 cm respectivement. Le volume de milieu contenu dans cette plaque est de 400L environ.

Les tubes (T1) sont conçues en verre feuilleté Saint Gobain avec un traitement de surface intérieur et extérieur par application du produit A.X.P. 1 commercialisé par la société MATT CHEM pour éviter l'accrochage, par exemple, des algues (« antifouling »). Le tube est maintenue entre deux pièces moulées faites dans un matériau neutre, c'est-à-dire en inox. La plaque est posée sur un profil recomposé soudé PRS en forme de cornière représenté sur la figure 7C (A). Elle comprend un large trou, c'est-à-dire de 20CM de diamètre à l'aplomb du tube pour permettre le passage des fluides. En partie haute, un élément en acier d'une épaisseur de 5 CM et d'une largeur de 3CM vient ceinturer la plaque pour l'empêcher de verser. La cornière (A) est fixée sur l'ossature primaire (O) par des tiges traversantes en acier.

La coursive (C) est une console en béton fabriqué qui est fixée dans l'ossature primaire (O) par des tiges traversantes.

La coursive (C) permet l'entretien des plaques par l'extérieur. Elle est suffisamment large, c'est-à-dire au minimum 70 cm pour permettre l'installation du tube et la circulation d'une personne chargée de la maintenance et du remplacement du tube le cas échéant.

Le tube (T1) peut être escamoté de la cornière (A) par l'intermédiaire d'un chariot de maintenance et d'un rail en partie haute qui lui sert de guide.

En particulier, le tube (T1) est disposé parallèlement, contre la façade (F) du bâtiment. Cette façade est un mur rideau avec prise en feuillure sur 4 côtés avec une alternance de vitrages (VI) Saint Gobain et de complexe isolant opaque (CI) formé d'une épaisseur d'isolant de 10CM coincé entre deux panneaux d'aluminium, devant la dalle pour assurer la sécurité incendie du bâtiment. L'ossature porteuse des façades murs rideaux est réalisée à partir de profilés carré en acier fixés par boulonnage sur l'ossature primaire en béton du bâtiment. L'habillage extérieur des montants et traverses est réalisé en profil tubulaire d'aluminium d'une section de 5 cm de large par 3 cm de profondeur. L'isolation entre l'extérieur et l'intérieur est assuré par un joint intercalaire Éthylène Propylène Diène Monomère (EPDM) commercialisé par la société Alder, d'une largeur de 9 mm. Les caractéristiques des vitrages (VI) peuvent être résumées comme telles : 10MM de verre extérieur de sécurité trempé - 14 mm vide - 12 mm verre de sécurité feuilleté intérieur, les verres provenant de la société Saint Gobain.

La protection solaire de ces façades est assurée par les coursives en béton qui assurent la fonction de brise soleil extérieur. Les tubes verticaux remplissent eux aussi un rôle de protection des rayons solaires incidents.

La plaque en verre est posée sur la cornière (A) devant la façade (F). La coursive (C) est dimensionnée de manière à permettre le passage et la station d'une personne devant le tube afin d'assurer sa maintenance et son remplacement le cas échéant. La largeur du passage est au minimum de 70 cm.

Afin de protéger la circulation d'une personne assurant la maintenance des tubes par l'extérieur, des garde-corps (GC) filant de 1 m de hauteur minimum sont fixés à la coursive par l'intermédiaire d'une entretoise. Une main courante formée d'une lisse en fer plat de 50 mm par 12mm est fixée au montant par une entretoise.

La pompe d'alimentation du milieu de culture est située dans les sous-sols du bâtiment avec les locaux techniques.

La pompe de pompage du milieu de culture est située dans les sous-sols du bâtiment avec les locaux techniques.

Des diodes électrochimiluminescentes émettant une longueur d'onde de 630 nm sont fixées par boulonnage sur une platine en acier à l'arrière des tubes sur la façade du bâtiment. L'allumage de ces diodes est contrôlé par une minuterie permettant l'allumage la nuit.

Un extracteur est disposé sur le conduit d'évacuation des effluents gazeux du bâtiment (Bat). Il s'agit d'un ventilateur permettant l'acheminement de l'effluent dans une gaine de 2 cm de diamètre au contenant de culture. Ce ventilateur est relié au dispositif de contrôle permettant ainsi d'augmenter et ou diminuer le débit de l'effluent en fonction de la concentration du CO₂ dans le milieu de culture.

Ainsi, on récupère le CO₂ du bâtiment pour l'injecter dans le dispositif de l'invention.

La culture des algues est effectuée dans le milieu précité et la biomasse récupérée par pompage du milieu dans le réservoir de stockage.

De manière surprenante, les inventeurs constatent que la disposition des plaques ou d'un contenant de culture devant la façade du bâtiment permet d'isoler celui-ci de l'air extérieur et d'augmenter son rendement énergétique.

L'apport de CO₂ et de NO₂ par l'effluent gazeux dans le milieu de culture contenant les algues en présence de soleil et/ou sous éclairage avec les diodes, permet de recycler le CO₂ et le NO₂ tout en produisant de la matière organique et d'émettre de l'oxygène par les algues. Ce recyclage est fait naturellement par les algues via le procédé de biosynthèse. Cet effluent permet en outre de chauffer le milieu de culture des algues.

De manière surprenante, les inventeurs constatent que la température moyenne de la culture est plus stable lorsque le contenant est situé devant la façade du bâtiment. Ainsi, les rendements de production et de purification de effluent gazeux sont également supérieurs.

Cet exemple démontre donc clairement que le dispositif de l'invention permet à la fois d'isoler le bâtiment mais également de recycler les effluents gazeux issus de bâtiments traités par le dispositif de la présente invention.

### Exemple 2 : Dispositif hors de l'invention disposé sur une façade de bâtiment produisant de la biomasse.

Dans cet exemple, on utilise le dispositif décrit dans l'exemple 1 pour former de la biomasse.

Dans ce cas, un extracteur est disposé sur le conduit d'évacuation des effluents gazeux d'une centrale thermique voisine du bâtiment (Bat).

Ainsi, on récupère le CO₂ d'une source externe du bâtiment pour l'injecter dans le dispositif de l'invention,

Les algues croissent plus rapidement avec cette addition de CO₂.

### Exemple 3 : Utilisation selon l'invention du dispositif disposé sur une façade d'un bâtiment avec une double peau en verre

La figure 2 représente le dispositif de l'exemple 1 disposé entre la façade d'un bâtiment (F) qui devient l'enveloppe intérieure et une sur-façade vitrée, cette dernière formant l'enveloppe extérieure (DP).

La figure 2 A est une vue de dessus, 2B est une vue de face et 2 C une section transversale.

Les algues, milieu de culture et conditions de culture sont identiques à ceux de l'exemple 1

Dans cet exemple, le contenant de culture est un tube vertical (T1). Ce tube est un tube en verre d'un diamètre de 40 cm. La taille des tubes est de 3.20 m tel que représenté sur la figure 2.

Des diodes électrochimiluminescentes (E) émettant une longueur d'onde de 630 nm sont fixées par boulonnage sur la façade du bâtiment. L'allumage de ces diodes est contrôlé par une minuterie permettant l'allumage la nuit.

L'enveloppe extérieure (DP) est un mur rideau avec prise en feuillure sur 4 côtés. L'ossature porteuse en acier des façades murs rideau est réalisée à partir de profilés carré de 20 cm de profondeur par 5 cm de large en acier fixés par boulonnage sur la coursive (C) en béton préfabriquée. La coursive est identique à celle de l'exemple 1.

L'habillage extérieur des montants et traverses est réalisé en profils tubulaires de 5 cm de large par 3 cm de profondeur d'aluminium. L'isolation entre l'extérieur et l'intérieur est assuré par un joint intercalaire Éthylène Propylène Diène Monomère (EPDM) commercialisé par la société Alder, d'une largeur de 9 mm. Les caractéristiques des vitrages (V) peuvent être résumées comme telles ; 10mm de verre extérieur de sécurité trempé - 14mm vide - 12mm verre de sécurité feuilleté intérieur, les verres provenant de la société Saint Gobain.

Le tube en verre (T1) est posé sur une cornière (A) devant la façade (F) du bâtiment. La cornière (A) est identique à celle de l'exemple 1. La coursive (C) est dimensionnée de manière à permettre le passage et la station d'une personne devant le tube afin d'assurer sa maintenance et son remplacement le cas échéant. La largeur de passage est au minimum de 70cm.

La circulation d'une personne assurant la maintenance des tubes se fait à l'intérieur de la double peau entre l'enveloppe intérieure et l'enveloppe extérieure vitrée (DP).

Cet exemple de réalisation comprenant une double peau est schématisé sur la figure 11 B qui représente une vue de dessus d'un bâtiment (Bat) avec une enveloppe extérieure (DB) et un espace (ES) dans lequel le dispositif est présent.

La double peau démontre de très bon résultat en termes d'isolation thermique car la ventilation du bâtiment se fait avec reprise d'air sur l'intérieur de la double peau qui reste elle-même à une température proche de celle des espaces intérieurs du bâtiment.

Elle permet de délimiter un espace dans lequel le contenant de culture est situé. Les inventeurs démontre donc de manière surprenante que cette double peau permet en outre de définir un espace dans lequel la température est régulée par les apports calorifiques du bâtiment. Cette enveloppe permet en outre d'isoler le contenant de culture de l'air extérieur et ainsi de régulariser les conditions de cultures des algues.

L'apport de CO₂ et de NO₂ par l'effluent gazeux dans le milieu de culture contenant les algues en présence de soleil et/ou sous éclairage avec les diodes, permet de recycler le CO₂ et le NO₂ tout en produisant de la matière organique et d'émettre de l'oxygène par les algues. Ce recyclage est fait naturellement par les algues via le procédé de biosynthèse.

La récupération de la biomasse et le renouvellement du milieu de culture est réalisé comme indiqué dans l'exemple 1.

Lors de la mise en oeuvre de cet exemple, les inventeurs constatent de manière surprenante que la température moyenne de la culture est plus stable lorsque le contenant est situé entre la façade (F) du bâtiment et l'enveloppe extérieure de la double peau (DP). Les rendements de production de la biomasse et de purification de l'effluent gazeux sont également supérieurs à des dispositifs en plein champs.

Cet exemple démontre donc clairement que la présente invention permet à la fois d'isoler le bâtiment mais également de recycler les effluent gazeux issus de bâtiments sont traités par le dispositif de la présente invention.

### Exemple 3 : Utilisation selon l'invention du dispositif disposé sur une façade d'un bâtiment avec une membrane en en éthylène tétrafluoroéthylène ETFE.

On décrit ici un mode de réalisation de l'invention. La figure 3 représente le dispositif de l'exemple 1 disposé entre la façade d'un bâtiment (F) qui devient l'enveloppe intérieure et une membrane en ETFE, cette dernière formant l'enveloppe extérieure. Le milieu de culture, l'algue, les conditions de culture, le bâtiment et la façade sont identiques à ceux de l'exemple 1. La figure 3 A est une vue de dessus, 3B est une vue de face et 3 C une section transversale.

Dans cet exemple, le contenant de culture est un tube vertical (T1). Ce tube est un tube en verre d'un diamètre de 40 CM. La taille des tubes est de 3.20 m tel que représenté sur la figure 2.

L'enveloppe extérieure en membrane ETFE est boulonné sur le nez de dalle de la coursive (C) par une structure faites de montants (XE) et de traverses (YE) en acier boulonnés entre eux. La membrane en ETFE est fixée sur la structure porteuse par l'intermédiaire de capots serreurs qui viennent serrer la membrane sur ses 4 côtés. Les capots serreurs sont réalisés en profils tubulaires de 5 cm de large par 3 cm de profondeur d'aluminium. L'isolation entre l'extérieur et l'intérieur est assuré par un joint intercalaire Éthylène Propylène Diène Monomère (EPDM) commercialisé par la société Alder, d'une largeur de 9 mm. La coursive est identique à celle de l'exemple 1.

Le tube en verre (T1) est posé sur une cornière (A) devant la façade (F) du bâtiment. La cornière (A) est identique à celle de l'exemple 1. La coursive (C) est dimensionnée de manière à permettre le passage et la station d'une personne devant le tube afin d'assurer sa maintenance et son remplacement le cas échéant. La largeur de passage est au minimum de 70 cm. La circulation d'une personne assurant la maintenance des tubes se fait à l'intérieur de la double peau entre l'enveloppe intérieure et la membrane en ETFE (ME).

Tel que démontré dans cet exemple, la membrane en ETFE permet en outre l'isolation thermique du bâtiment et permet d'isoler le contenant de culture de l'air extérieur.

### Exemple 4 : Dispositif selon l'invention disposé sur une toiture d'un bâtiment

On décrit ici un mode de réalisation de l'invention. Le dispositif utilisé est schématisé sur la figure 4.

Le milieu de culture, l'algue, les conditions de culture, le bâtiment et la façade sont identiques à ceux de l'exemple 1.

Dans cet exemple, les tubes sont fixés par vissage sur la dalle du dernier plancher du bâtiment. Les tubes sont maintenus entre eux en partie haute par des poutres en acier.

La toiture (TO) en verre, renforce l'isolation thermique du bâtiment et permet d'isoler le tube de culture de l'air extérieur.

### Exemple 5 : Dispositif avec un contenant de plaque fixé sur une façade de bâtiment

On décrit ici un mode de réalisation de l'invention. Le dispositif utilisé est schématisé sur la figure 5. Le milieu de culture, l'algue, les conditions de culture, le bâtiment et la façade sont identiques à ceux de l'exemple 1.

Dans cet exemple, le contenant de culture est un panneau creux vertical (T2). Ce panneau creux est constitué de verre parallèle de 5 CM d'épaisseur profilés avec des angles arrondis en verre profilé qui relient les deux plaques entre elles. La partie profilée en verre de la plaque à une profondeur de 20 cm et une largeur de 90 cm. Elle a une hauteur de 3,20 m.

Le panneau creux (T2) est posé sur une pièce rectangulaire moulée de 100 cm de large et de 30 cm de profondeur. Cette pièce fait tenir le verre par cerclage avec un la pièce moulée en partie basse et en partie haute, c'est-à-dire à une hauteur de 3 cm Et de 320 cm respectivement. Le volume de milieu contenu dans cette plaque est de 500L environ.

Dans cet exemple, lorsque le contenant de culture est disposé contre la façade, il est fixé de la même manière que l'exemple 1.

### Exemple 6 : Dispositif avec un contenant de culture circulaire fixé sur une façade de bâtiment

On décrit ici un mode de réalisation de l'invention. Le dispositif utilisé est schématisé sur la figure 6. Le milieu de culture, l'algue, les conditions de culture, le bâtiment et la façade sont identiques à ceux de l'exemple 1.

Dans cet exemple, le contenant de culture est un tube vertical (T3). Ce tube est un tube multicouche comprenant un tube extérieur (T3ext) en polycarbonate d'une épaisseur de 5 cm, un tube intermédiaire en verre d'une épaisseur de 5 cm (T3int) et un tube intérieur d'une épaisseur de 5 cm (Ti) en verre représenté sur la figure 10 B.

Les tubes ont une hauteur de 3,20 m dans la figure 6, le tube vitré extérieur a un diamètre de 50 cm, le tube intérieur a un diamètre de 10 cm. Le tube (T3) est posé sur une pièce carré moulée de 60cm de côté. Cette pièce fait tenir le verre par cerclage avec un la pièce moulée en partie basse et en partie haute, c'est-à-dire à une hauteur de 3 cm et de 320 cm respectivement. Le volume de milieu contenu dans cette plaque est de 400L environ.

Le tube interne (TI) comprend des diodes électrochimilunescentes (E) émettant une longueur d'onde de 630 nm.

Les tubes sont éclairés automatiquement par ces diodes la nuit grâce à un système de minuterie automatique.

Le milieu de culture est compris entre la surface extérieure du tube intérieur et la surface interne du tube intermédiaire.

Dans cet exemple, lorsque le contenant de culture est disposé contre la façade, il est fixé de la même manière que l'exemple 1.

### Exemple 7 : Production et transformation de biomasse par un dispositif selon l'invention disposé sur une façade d'un bâtiment avec une double peau.

Les conditions de culture et milieu utilisé dans cet exemple sont identiques à celles de l'exemple 6.

Dans cet exemple l'effluent gazeux comprenant en outre du CO₂ et du NO₂ est récupérer d'un circuit de ventilation d'un bâtiment. Il est acheminé à un ensemble de tubes verticaux multicouches d'une hauteur de 2 m et de 80 cm de diamètre. Les tubes comprennent un tube extérieur en polycarbonate, un tube intermédiaire en verre et un tube intérieur en verre et un socle carré en haut et en bas du tube comme présenté sur la figure 6.

Le tube multicouche comprend entre le tube intermédiaire et le tube interne un milieu de culture identique à celui de l'exemple 1 et des algues Chlorella. L'effluent gazeux est injecté dans le milieu par l'extrémité inférieur du tube via un tuyau souple en polyéthylène. Le tube multicouche comprend dans le tube interne des diodes lumineuses émettant une lumière d'une longueur d'onde de 660 nm. Les tubes sont éclairés automatiquement par ces diodes la nuit grâce à un système de minuterie automatique.

Les tubes sont disposés devant une façade d'un bâtiment comme présenté dans l'exemple 1.

Un réservoir d'alimentation comprenant du milieu de culture est connecté aux tubes multicouches via un orifice situé à la base du contenant. Une pompe de vidange est également connectée au contenant avec un tube plastique par un orifice situé à la base du tube. L'ensemble est connecté indépendamment aux tubes permettant ainsi la vidange et le renouvellement du milieu de culture comme indiqué dans l'exemple 1 précité.

L'apport de CO₂ et de NO₂ par l'effluent gazeux dans le milieu de culture est réalisé comme décrit dans l'exemple 2 ci-dessus.

Les tubes contenant les algues en présence de soleil et/ou sous éclairage avec les diodes, permet de produire de la matière organique et l'émission d'oxygène par les algues. Cette production de matière est faite par photosynthèse par lesdites algues. La matière organique produite est récupérée par pompage de 75% du milieu de culture dans un contenant de stockage, à savoir une cuve de 100 Litres, tous les quatre jours.

Le milieu de culture pompé est ensuite soumis à une étape de transformation dans laquelle les triglycérides sont extraits. Ces triglycérides sont ensuite soumis à une réaction de trans-estérification avec du méthanol. Le produit obtenu est du monoesterméthylique correspondant à du biodiesel.

Dans un autre mode de réalisation, les algues sont prélevées avec le milieu de culture et les algues sont pressées à froid afin de récupérer une plus grande quantité de tryglycérides et donc de produire du biodiesel.

Dans un autre mode de réalisation, le milieu de culture pompé est acheminé à une centrale thermique à biomasse où il est consommé afin de produire de l'énergie.

Dans un autre mode de réalisation de cet exemple, le milieu de culture est soumis à une pyrolyse à une température comprise entre 50 et 500°C permettant de récupérer des hydrocarbures qui peuvent être utilisé comme biocarburant. La figure 6 représente un schéma de cet exemple.

Comme démontré dans les exemples précédents et dans cet exemple, la disposition du dispositif devant toute façade de bâtiment permet une régulation de la température autour de contenant de culture et permet d'isoler le bâtiment. En effet, les inventeurs ont constaté de manière surprenante que la façade du bâtiment a un apport calorifique sur l'air ambiant et qu'il permet de réguler la température de culture des algues. Par ailleurs, cette régulation permet en outre une épuration d'effluent gazeux et la production de biomasse.

Cet exemple démontre donc clairement que l'utilisation du dispositif sur une façade de bâtiment permet de produire de la matière organique par des algues tout en recyclant les effluents gazeux du bâtiment et en isolant celui-ci. La présente invention permet d'effectuer un pont énergétique entre le bâtiment et le dispositif de culture.

## Revendications

1. Utilisation d'un dispositif pour le traitement d'un effluent et/ou la fabrication d'une biomasse comprenant :
- un contenant de culture d'algues et/ou de microorganismes en milieu aqueux,
- un moyen d'alimentation de la culture d'algues et/ou de microorganismes
- un moyen d'injection dans la culture d'algues et/ou de microorganismes d'un effluent, ledit effluent provenant d'un bâtiment,
- un moyen de régulation de la température de la culture d'algues et/ou de microorganismes,
- éventuellement un éclairage favorable à la culture d'algues et/ou de microorganisme, et
- un moyen de récupération dudit effluent permettant de récupérer ledit effluent issu d'un bâtiment pour les injecter dans la culture d'algues et/ou de microorganismes,
dans lequel le contenant de culture d'algues et/ou de microorganismes est positionné entre deux surfaces : une surface de bâtiment et une surface extérieure formant une double peau, et
ledit effluent provient du bâtiment possédant la double peau et/ou d'un autre bâtiment.

2. Utilisation selon la revendication 1, dans lequel l'effluent est un effluent gazeux ou liquide.

3. Utilisation selon l'une quelconque des revendications précédentes, dans laquelle le contenant est un contenant transparent à la lumière à laquelle est sensible l'algue pour sa culture, et comprend en outre un moyen d'éclairage ou rétroéclairage.

4. Utilisation selon l'une quelconque des revendications précédentes dans laquelle le contenant de culture d'algues et/ou de microorganismes se présente sous une forme choisie dans le groupe comprenant un cylindre, un tube, un tube plat, un tube ondulé sur sa longueur, un panneau creux, une sphère, un cube, un parallélépipède rectangle, un parallélépipède rectangle avec des bord arrondis, une spirale, un sachet, une forme creuse sans arrête vive.

5. Utilisation selon l'une quelconque des revendications précédentes dans laquelle le dispositif comprenant en outre un moyen de chauffage de la culture d'algues et/ou de microorganismes choisi dans le groupe comprenant un moyen de récupération de la chaleur d'un bâtiment, un moyen de récupération de la chaleur extérieure, un moyen de récupération de la chaleur solaire, un moyen de récupération de l'énergie calorifique.

6. Utilisation selon l'une quelconque des revendications précédentes dans laquelle le dispositif comprenant en outre un moyen de refroidissement de la culture d'algues et/ou de microorganismes choisi dans le groupe comprenant un moyen de récupération de la fraicheur d'un bâtiment, un moyen de récupération de la fraicheur extérieure, un moyen de récupération de l'énergie calorifique.

7. Utilisation selon la revendication 5, dans laquelle le moyen de récupération de la chaleur d'un bâtiment est une double peau du bâtiment définie par ladite surface de bâtiment et ladite surface extérieure.

8. Utilisation selon la revendication 7, dans laquelle le moyen de récupération de la fraicheur d'un bâtiment est une double peau du bâtiment définie par ladite surface de bâtiment et ladite surface extérieure.

9. Utilisation selon l'une quelconque des revendications précédentes, dans laquelle le dispositif comprend en outre un renfort de structure permettant de supporter le contenant de culture d'algues et/ou de microorganismes, ledit renfort pouvant être fixé au bâtiment.

10. Utilisation selon l'une quelconque des revendications précédentes, ledit dispositif comprenant en outre au moins une coursive, ladite coursive pouvant servir de support du contenant de culture d'algues et/ou de microorganismes et/ou de moyen d'accès au contenant de culture d'algues et/ou de microorganismes pour du personnel d'entretien.

11. Utilisation selon l'une quelconque des revendications précédente dans laquelle la biomasse est une biomasse oléagineuse, une biomasse transformable en charbon ou en biopétrole,.ou une biomasse transformable en composés chimiques ou pharmaceutiques.

12. Utilisation selon l'une quelconque des revendications précédentes dans laquelle le dispositif est intégré à la structure d'un bâtiment ou rapporté sur le bâtiment.

13. Utilisation selon l'une quelconque des revendications précédentes, dans laquelle le bâtiment est un bâtiment choisi parmi un bâtiment industriel et/ou de bureaux et/ou d'habitation et/ou agricole et/ou un ouvrage d'art et/ou mixte.

14. Utilisation selon l'une quelconque des revendications précédentes, dans laquelle ledit dispositif comprend en outre un moyen de récupération de la biomasse formée par culture des algues et/ou microorganismes.

15. Utilisation selon l'une quelconque des revendications précédentes, dans laquelle ledit dispositif comprenant en outre un système de vidange du contenant de culture des algues.

16. Utilisation selon l'une quelconque des revendications précédentes, dans laquelle ledit dispositif comprenant en outre un ou plusieurs des moyens de contrôle et régulation de la culture des algues suivants : moyens de contrôle de l'alimentation des algues et/ou microorganisme, moyens de contrôle de l'injection de l'effluent à traiter, moyens de contrôle de la température, moyens de contrôle du pH, moyens de contrôle de l'éclairage des algues et/ou microorganismes.

17. Utilisation selon l'une quelconque des revendications précédentes dans laquelle le contenant de culture a une inclinaison comprise entre 0 et 90°

## Claims

1. Use of a device for treating an effluent and/or manufacturing a biomass, comprising:
- a container for culturing alga and/or microorganisms in an aqueous medium,
- a means of feeding the alga and/or microorganisms culture,
- a means of injecting an effluent into the alga and/or microorganisms culture, said effluent coming from a building,
- a means of regulating the temperature of the alga and/or microorganisms culture,
- optionally lighting favourable to the culture of algae and/or microorganisms, and
- a means of recovering said gaseous effluent issuing from a building in order to inject it into the alga and/or microorganism culture
wherein the container for culturing alga and/or microorganisms is between two surfaces: a building surface and an exterior surface forming a double skin, and said effluent coming from the building having the double skin and/or from another building.

2. Use according to claim 1, wherein the effluent is a gaseous or liquid effluent.

3. Use according to any one of the preceding claims, wherein the container is a container transparent to light to which the alga is sensitive for culture thereof, and further comprise a lighting or backlighting means.

4. Use according to any one of the preceding claims, in which the alga and/or microorganism culture container is in a form selected from the group comprising a cylinder, a tube, a flat tube, a tube corrugated over its length, a hollow panel, a sphere, a cube, a right-angled parallelepiped, a right-angled parallelepiped with rounded edges, a spiral, a sachet or a hollow shape without sharp edge.

5. Use according to any one of the preceding claims, wherein the device further comprises a means of heating the alga and/or microorganism culture selected from the group comprising a means of recovering heat from a building, a means of recovering external heat, a means of recovering solar heat and a means of recovering heat energy.

6. Use according to any one of the preceding claims, wherein the device further comprises a means of cooling the alga and/or microorganism culture selected from the group comprising a means of recovering coolness from a building, a means of recovering external coolness and a means of recovering heat energy.

7. Use according to claim 5, wherein the means of recovering heat from a building is a double skin of the building defined by said surface of building and said exterior surface.

8. Use according to claim 7, wherein the means of recovering coolness from a building is a double skin of the building defined by said surface of building and said exterior surface.

9. Use according to any one of the preceding claims, wherein the device also comprises a structure reinforcement for supporting the alga and/or microorganism culture container, said reinforcement being able to be fixed to the building.

10. Use according to any one of the preceding claims, said device further comprising at least one gangway, said gangway being able to serve as a support for the alga and/or microorganism culture container and/or as a means of access to the alga and/or microorganism culture container for maintenance personnel.

11. Use according to any one of the preceding claims, wherein the biomass is an oily biomass, a biomass that can be converted into coal or biopetroleum, or a biomass that can be converted into chemical or pharmaceutical compounds.

12. Use according to any one of the preceding claims, wherein which the device is integrated in the structure of a building or attached to the building.

13. Use according to any one of the preceding claims, wherein the building is an industrial and/or office and/or dwelling and/or agricultural building and/or a civil engineering and/or mixed structure.

14. Use according to any one of the preceding claims, wherein the device further comprises a means of recovering biomass formed by the culture of algae and/or microorganisms.

15. Use according to any one of the preceding claims, said device also comprises a system of draining the alga culture container.

16. Use according to any one of the preceding claims, wherein said device also comprising one or more of the following means of controlling and regulating the culture of alga: means of controlling the supply of algae and/or microorganisms, means of controlling the injection of effluent to be treated, means of controlling the temperature, means of controlling the pH and means of controlling the lighting of the algae and/or microorganisms.

17. Use according to any one of the preceding claims, wherein the culture container has and inclination between 0 and 90°.

## Patentansprüche

1. Verwendung einer Vorrichtung zur Behandlung von Abflüssen und/oder zur Herstellung einer Biomasse umfassend:
- einen Behälter für die Kultur von Algen und/oder Mikroorganismen in einem wässrigen Medium,
- ein Mittel zum Nähren der Algen- und/oder der Mikroorganismenkultur,
- ein Mittel zum Einspritzen von Abflüssen in die Algen- und/oder Mikroorganismenkultur, wobei die Abflüsse von einem Gebäude stammen,
- ein Mittel zur Regulierung der Temperatur der Algen- und/oder Mikroorganismenkultur,
- gegebenenfalls eine der Algen- und/oder Mikroorganismenkultur förderliche Beleuchtung, und
- ein Mittel zur Rückgewinnung der Abflüsse, das es ermöglicht, die Abflüsse eines Gebäudes zu sammeln, um sie in die Algen- und/oder Mikroorganismenkultur einzuspritzen,
in welcher der Behälter für die Algen- und/oder Mikroorganismenkultur zwischen zwei Oberflächen angebracht ist: eine Gebäudeoberfläche und eine Außenoberfläche, die eine zweite Haut bildet, und
wobei die Abflüsse von dem Gebäude mit der doppelten Haut und/oder von einem anderen Gebäude kommen.

2. Verwendung nach Anspruch 1, **dadurch gekennzeichnet, dass** die Abflüsse Abgase oder Abwässer sind.

3. Verwendung nach einem beliebigen der vorstehenden Ansprüche, wobei der Behälter ein für das Licht durchlässiger Behälter ist, für das die Algenkultur sensibel ist, und ferner ein Beleuchtungs- oder Hintergrundbeleuchtungsmittel umfasst.

4. Verwendung nach einem beliebigen der vorstehenden Ansprüche, in welcher der Behälter für die Algen- und/oder Mikroorganismenkultur eine Form aufweist, die aus der Gruppe ausgewählt ist, die einen Zylinder, ein Rohr, ein flaches Rohr, ein längsgewelltes Rohr, eine hohle Platte, eine Kugel, einen Kubus, ein rechteckiges Parallelepiped, ein rechteckiges Parallelepiped mit abgerundeten Kanten, eine Spirale, eine Tasche, eine Hohlform ohne scharfe Kante umfasst.

5. Verwendung nach einem beliebigen der vorstehenden Ansprüche, in welcher die Vorrichtung ferner ein Mittel zum Erwärmen der Algen- und/oder Mikroorganismenkultur umfasst, aus der Gruppe ausgewählt, die ein Mittel zur Rückgewinnung der Wärme eines Gebäudes, ein Mittel zur Rückgewinnung der Außenwärme, ein Mittel zur Rückgewinnung der Sonnenwärme, ein Mittel zur Rückgewinnung der Wärmeenergie umfasst.

6. Verwendung nach einem beliebigen der vorstehenden Ansprüche, in welcher die Vorrichtung ferner ein Mittel zum Kühlen der Algen- und/oder Mikroorganismenkultur umfasst, aus der Gruppe ausgewählt, die ein Mittel zur Rückgewinnung der Kühle eines Gebäudes, ein Mittel zur Rückgewinnung der Außenkühle, ein Mittel zur Rückgewinnung der Wärmeenergie umfasst.

7. Verwendung nach Anspruch 5, wobei das Mittel zur Rückgewinnung der Wärme eines Gebäudes eine doppelte Haut des Gebäudes ist, die durch die Gebäudeoberfläche und die Außenoberfläche definiert ist.

8. Verwendung nach Anspruch 7, wobei das Mittel zur Rückgewinnung der Kühle eines Gebäudes eine doppelte Haut des Gebäudes ist, die durch die Gebäudeoberfläche und die Außenoberfläche definiert ist.

9. Verwendung nach einem beliebigen der vorstehenden Ansprüche, wobei die Vorrichtung ferner eine Verstärkung der Struktur umfasst, die es ermöglicht, den Behälter für die Algen- und/oder Mikroorganismenkultur zu tragen, wobei die Verstärkung am Gebäude fixiert werden kann.

10. Verwendung nach einem beliebigen der vorstehenden Ansprüche, wobei die Vorrichtung ferner mindestens eine Galerie umfasst, wobei die Galerie als Mittel zum Tragen des Algen- und/oder Mikroorganismenkulturbehälters und/oder als Mittel zum Zugang zu dem Algen- und/oder Mikroorganismenkulturbehälter für das Wartungspersonal dienen kann.

11. Verwendung nach einem beliebigen der vorstehenden Ansprüche, wobei die Biomasse eine ölhaltige Biomasse, eine in Kohle und oder in Biopetroleum oder in chemische oder pharmazeutische Verbindungen umwandelbare Biomasse ist.

12. Verwendung nach einem beliebigen der vorstehenden Ansprüche, wobei die Vorrichtung in die Gebäudestruktur integriert oder an das Gebäude angebracht wird.

13. Verwendung nach einem beliebigen der vorstehenden Ansprüche, wobei das Gebäude unter folgenden gewählt wird: ein Industriegebäude und/oder Bürogebäude und/oder Wohngebäude und/oder Landwirtschaftsgebäude und/oder Ingenieurbauwerk und/oder eine Mischform.

14. Verwendung nach einem beliebigen der vorstehenden Ansprüche, wobei die Vorrichtung ferner ein Mittel zur Rückgewinnung der von der Algen- und/oder Mikroorganismenkultur gebildeten Biomasse umfasst.

15. Verwendung nach einem beliebigen der vorstehenden Ansprüche, wobei die Vorrichtung ferner ein System zur Entleerung des Behälters für die Algen- und/oder Mikroorganismenkultur umfasst.

16. Verwendung nach einem beliebigen der vorstehenden Ansprüche, wobei die Vorrichtung ferner ein oder mehrere der folgenden Mittel zur Überwachung und Regulierung der Kultur der Algen und/oder Mikroorganismen umfasst : Mittel zur Überwachung der Nährmittel der Algen und/oder Mikroorganismen, Mittel zur Überwachung der Einspritzung des zu behandelnden Abflusses, Mittel zur Überwachung der Temperatur, Mittel zur Überwachung des pH-Werts, Mittel zur Überwachung der Beleuchtung der Algen und/oder Mikroorganismen.

17. Verwendung nach einem beliebigen der vorstehenden Ansprüche, wobei der Kulturbehälter eine Neigung zwischen 0 und 90 ° aufweist.
